# EUROPEAN PATENT APPLICATION

(11) **EP 4 484 416 A1**
(43) Date of publication of application: **01.01.2025**
(21) Application number: 23759386.8
(22) Date of filing: 16.02.2023
(51) Int. Cl.: C07D 307/91, H10K 50/15, H10K 50/16, H10K 50/17, H10K 50/18, H10K 59/10, H10K 85/60

(54) **ARYLAMINE COMPOUND, ORGANIC ELECTROLUMINESCENCE ELEMENT AND ELECTRONIC DEVICE**

(30) Priority: 24.02.2022 KR 20220024675
(71) Applicant: Hodogaya Chemical Co., Ltd., Tokyo, 105-0021 (JP)
(72) Inventor: KASE, Kouki, Tokyo (JP); KO, Sang-Won, Tokyo (JP); LIM, Jae-Geon, Tokyo (JP); IZUMIDA, Junichi, Tokyo (JP); HAYASHI, Shuichi, Tokyo (JP)
(74) Representative: dompatent von Kreisler Selting Werner - Partnerschaft von Patent- und Rechtsanwälten mbB
(86) International application number: PCT/IB2023/051389
(87) International publication number: WO 2023/161767

(57) **Abstract**

The objective of the present invention is to provide an organic compound having excellent properties as a material for a high-efficiency, high-durability organic EL device, such as exhibiting good hole injection and transport performance, having electron blocking ability and being highly stable in a thin film state, and to provide a high-efficiency, high-durability organic EL device using the compound. The arylamine compound of the present invention has excellent heat resistance and good hole transport ability. The organic EL device using the compound in the hole transport layer, electron blocking layer, light emitting layer and hole injection layer of the organic EL device exhibited good device properties.

## Description

### [Technical Field]

The present disclosure relates to a compound suitable for an organic electroluminescence element (hereinafter abbreviated as an organic EL element), which is a self-luminous element preferable for various display devices, and an element, and specifically to an arylamine compound and an organic EL element using the compound.

### [Background Art]

Since organic EL elements are self-luminous elements, active research has been conducted on them in that they are brighter than a liquid crystal device, have excellent visibility, and enable clear displays.

In 1987, C. W. Tang and others at Eastman-Kodak made organic EL elements using organic materials practical by developing a layered structure element in which various roles were assigned to each material. They stack a phosphor capable of transporting electrons and an organic material capable of transporting holes, and inject charges of both directions into the phosphor layer to emit light, thereby obtaining high luminance of 1000 cd/m2 or more at a voltage of 10 V or less (e.g., see Patent Document 1 and Patent Document 2).

To date, many improvements have been made for the practical use of organic EL elements, and various roles of the laminated structure have been further subdivided, so that high efficiency and durability have been achieved by an electroluminescent element in which the anode, hole injection layer, hole transport layer, light emitting layer, electron transport layer, electron injection layer, and cathode are sequentially formed on the substrate (e.g., see Non-Patent Document 1).

In addition, for the purpose of further improving luminous efficiency, the use of triplet excitons has been attempted, and the use of phosphorescent compounds has been examined (e.g., see Non-Patent Document 2). In addition, elements using light emission by thermally activated delayed fluorescence (TADF) have also been developed, and in 2011, Adachi et al. of Kyushu University realized an external quantum efficiency of 5.3% with an element using a thermally activated delayed fluorescent material (e.g., see Non-Patent Document 3).

The light emitting layer may also be manufactured by doping a charge-transporting compound, generally called a host material, with a fluorescent compound, a phosphorescent compound, or a material that emits delayed fluorescence. As described in the above-mentioned non-patent document, the selection of organic materials in organic EL elements has a great influence on various characteristics such as efficiency and durability of the elements (e.g., see non-patent document 2).

In organic EL elements, charges injected from both electrodes recombine in the light emitting layer so that light emission is obtained, but since it is important how to efficiently transfer the positive charges of holes and electrons to the light emitting layer, it is necessary to do it with an element having excellent carrier balance. Therefore, a material that has the characteristics of increasing the hole injection properties of supplying holes injected from the anode to the light emitting layer and increasing the electron blocking properties of blocking the electrons injected from the cathode may be used, thereby increasing the probability that holes and electrons are recombined in the light emitting layer, and furthermore, the excitons generated within the light emitting layer may be confined, thereby obtaining high luminous efficiency. To this end, the role played by hole transport materials is important, and hole transport materials with high hole injection properties, high hole mobility, high electron blocking properties, and further high durability against electrons are required.

In addition, the heat resistance and amorphous properties of the materials are also important regarding the lifespan of the element. In materials with low heat resistance, thermal decomposition occurs even at low temperatures due to the heat generated when the element is driven, causing the materials to deteriorate. In materials with low amorphous properties, crystallization of the thin film occurs even in a short period of time, causing the element to deteriorate. For that reason, the materials used are required to have high heat resistance and good amorphous properties.

Hole transport materials that have been used so far in organic EL elements include N,N'-diphenyl-N,N'-di(α-naphthyl)benzidine (NPD) and various aromatic amine derivatives (e.g., see Patent Document 1 and Patent Document 2). However, although NPD has a good hole transport ability, since its glass transition point (Tg), which is an indicator of heat resistance, is low at 96°C, the element characteristics are deteriorated due to crystallization under high temperature conditions (e.g., see Non-Patent Document 4).

In addition, there are compounds with excellent hole mobility of 10⁻³ cm²/Vs or more among the aromatic amine derivatives described in the above patent documents (e.g., see Patent Document 1 and Patent Document 2), but since the electron blocking properties are insufficient, some of the electrons escape the light emitting layer, no improvement in luminous efficiency can be expected. In order to further increase efficiency, materials with higher electron blocking properties, more stable thin films, and higher heat resistance have been required. In addition, there have been reports of highly durable aromatic amine derivatives (e.g., see Patent Document 3), but there have been no examples of their use as organic EL elements as ones used as charge transport materials used in electrophotographic photoreceptors.

To solve this problem, although substituted carbazole structures and arylamine compounds have been proposed as compounds with improved properties such as heat resistance and hole injection properties (e.g., see Patent Document 4 and Patent Document 5), since element lifespan and luminous efficiency have been improved in elements using these compounds in the hole injection layer or the hole transport layer, but this cannot be said to be sufficient yet, additional low driving voltage, high emission efficiency, and longer element lifespan have been required.

### [Prior Art Documents]

### [Patent Documents]

(Patent Document 1) US Patent No. 5792557 Specification
(Patent Document 2) US Patent No. 5639914 Specification
(Patent Document 3) US Patent No. 7759030 Specification
(Patent Document 4) JP4589223B2
(Patent Document 5) JP6674892B2
(Patent Document 6) European Patent No. 2684932 Specification
(Patent Document 7) JP6748335B1
(Patent Document 8) KR102288034B1

### [Non-Patent Documents]

(Non-Patent Document 1) The Japanese Journal of Applied Physics 9th Lecture Preliminary Book, pages 55 to 61 (2001)
(Non-Patent Document 2) The Japanese Journal of Applied Physics 9th Lecture Preliminary Book, pages 23 to 31 (2001)
(Non-Patent Document 3) Appl. Phys. Let., 98, 083302 (2011)
(Non-Patent Document 4) Organic EL Forum 3rd Annual Meeting Preview, pages 13 to 14 (2006)

### [Disclosure]

### [Technical Problem]

An object of the present disclosure is to provide as a material for an organic EL element with high efficiency and high durability, a material for an organic EL element which (1) has excellent hole injection and transport performance, (2) has electron blocking ability, (3) has high stability in a thin film state, and (4) has excellent durability.

It is to provide an organic EL element that (1) has high luminous efficiency and power efficiency, (2) has low luminescence start voltage and practical driving voltage, and (3) has a long lifespan by using the material of the present disclosure.

### [Technical Solution]

In order to achieve the above object, the present inventors focused on the fact that arylamine compounds have excellent hole injection and transport abilities, thin film stability and durability, and pursued the introduction of phenanthrenyl groups and naphthylene groups and optimization of substituents, thereby dramatically improving the properties of the material. Even in organic EL elements, the performance of luminous efficiency and power efficiency has been improved, the luminous emission starting voltage and practical driving voltage have been enabled to be suppressed, and a longer lifespan exceeding the conventional lifespan has been realized. As a result, the present disclosure has been completed.
1) That is, the present disclosure is an arylamine compound represented by the general formula (a) or (b) below.

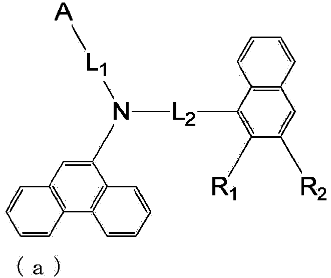

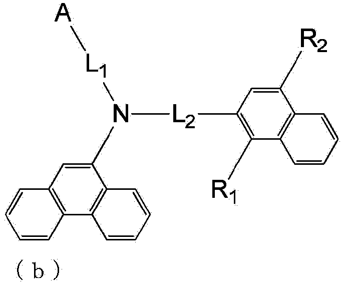

In the general formula (a) or (b), A indicates a substituted or unsubstituted dibenzofuranyl group, or a substituted or unsubstituted dibenzothienyl group,

L₁ indicates a divalent group obtained by removing two hydrogen atoms from unsubstituted benzene, or a divalent group obtained by removing two hydrogen atoms from unsubstituted biphenyl,

L₂ indicates a divalent group obtained by removing two hydrogen atoms from unsubstituted benzene, and

R₁ and R₂ each indicate a hydrogen atom, a substituted or unsubstituted aromatic hydrocarbon group, a substituted or unsubstituted aromatic heterocyclic group, or a substituted or unsubstituted condensed polycyclic aromatic group, which may be the same or different.

2) In addition, the present disclosure is an arylamine compound described in 1) above, represented by the general formula (c), (d), (e), or (f) below.

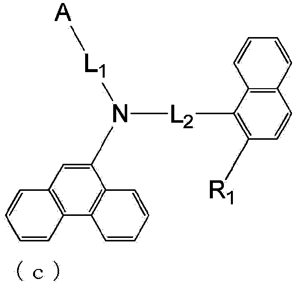

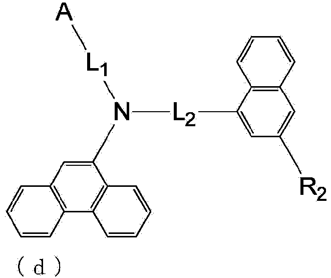

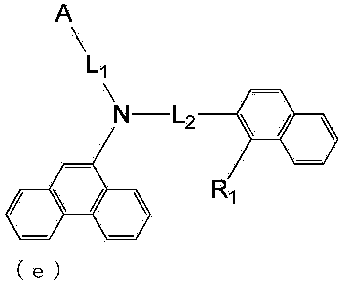

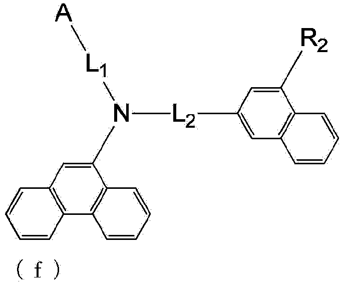

In the general formula (c), (d), (e), or (f), A, L₁, L₂, R₁, and R₂ are the same definitions as in the general formula (a) or (b).

3) In addition, the present disclosure, in the general formula (c), (d), (e), or (f) above,
is an arylamine compound described in 1) or 2) above, wherein L₂ is represented by a 1,4-phenylene group.

4) In addition, the present disclosure, in the general formula (c), (d), (e), or (f) above,
in which A is represented by an unsubstituted dibenzofuranyl group or an unsubstituted dibenzothienyl group,
is an arylamine compound described in any one of items 1) to 3) above.

5) In addition, the present disclosure, in the general formula (c), (d), (e), or (f) above,
is an arylamine compound described in any one of items 1) to 4) above, wherein A is represented by an unsubstituted dibenzofuranyl group.

6) In addition, the present disclosure, in the general formula (c), (d), (e), or (f) above,
is an arylamine compound described in any one of items 1) to 5) above, wherein A is represented by an unsubstituted 3-dibenzofuranyl group or an unsubstituted 4-dibenzofuranyl group.

7) In addition, the present disclosure is an organic EL element having a pair of electrodes and at least one organic layer sandwiched between them, wherein the organic layer contains the arylamine compound described in any one of 1) to 6) above.

8) In addition, the present disclosure is an organic EL element described in 7) above, wherein the organic layer is a hole transport layer.

9) In addition, the present disclosure is an organic EL element described in 7) above, wherein the organic layer is an electron blocking layer.

10) In addition, the present disclosure is an organic EL element described in 7) above, wherein the organic layer is a hole injection layer.

11) In addition, the present disclosure is an organic EL element described in 7) above, wherein the organic layer is a light emitting layer.

12) In addition, the present disclosure is an electronic device having a pair of electrodes and at least one organic layer sandwiched between them, wherein the organic layer contains the arylamine compound described in any one of 1) to 6) above.

Represented by R₁ and R₂ in the general formula (a) or (b)
in ^{┌}substituted or unsubstituted aromatic hydrocarbon group_{┘} , ^{┌}substituted or unsubstituted aromatic heterocyclic group_{┘} , or ^{┌}substituted or unsubstituted condensed polycyclic aromatic group_{┘} ,
an ^{┌}aromatic hydrocarbon group_{┘} , ^{┌}aromatic heterocyclic group_{┘} , or ^{┌}condensed polycyclic aromatic group_{┘} may include,
specifically, an aryl group having 6 to 30 carbon atoms, a heteroaryl group having 2 to 20 carbon atoms, etc. in addition to a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a fluorenyl group, a spirofluorenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, an azafluorenyl group, a diazafluorenyl group, an azaspirobifluorenyl group, a diazaspirobifluorenyl group, a quinoxalinyl group, a benzoimidazolyl group, a pyrazolyl group, a dibenzofuranyl group, a dibenzothienyl group, a naphthyridinyl group, a phenanthrolinyl group, an acridinyl group, and a carbolinyl group.

Represented by R₁ and R₂ in the general formula (a) or (b)
a ^{┌}substituent_{┘} in a ^{┌}substituted aromatic hydrocarbon group_{┘} , ^{┌}substituted aromatic heterocyclic group_{┘} , or ^{┌}substituted condensed polycyclic aromatic group_{┘} may include,
specifically, deuterium atom, cyano group, nitro group; halogen atoms such as fluorine atom, chlorine atom, bromine atom, and iodine atom; silyl groups such as trimethylsilyl group and triphenylsilyl group; straight-chain or branched alkyl groups having 1 to 6 carbon atoms, such as a methyl group, an ethyl group, and a propyl group; straight-chain or branched alkyloxy groups having 1 to 6 carbon atoms, such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups or condensed polycyclic aromatic groups such as a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a fluorenyl group, and a spirofluorenyl group; and aromatic heterocyclic groups such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzoimidazolyl group, a pyrazolyl group, a dibenzofuranyl group, a dibenzothienyl group, and a carbolinyl group, and these substituents may further be substituted by the substituents exemplified above. Further, the benzene rings substituted with these substituents, or the substituents multiple substituted with the same benzene ring, may be bonded to each other to form a ring by interposing a single bond, a substituted or unsubstituted methylene group, an oxygen atom, or a sulfur atom.

The ^{┌}substituent_{┘} in the ^{┌}substituted dibenzofuranyl group_{┘} or ^{┌}substituted dibenzothienyl group_{┘} represented by A in the general formula (a) or (b) may include,
represented by R₁ and R₂ in the general formula (a) or (b),
substituents that are the same as those shown as ^{┌}substituent_{┘} in the ^{┌}substituted aromatic hydrocarbon group_{┘} , ^{┌}substituted aromatic heterocyclic group_{┘} , or ^{┌}substituted condensed polycyclic aromatic group_{┘} , and the aspects that can be taken may also include the same ones.

L₁ in the general formula (a) or (b) is preferably a phenylene group is preferable, and more preferably a 1,4-phenylene group.

A in the general formula (a) or (b) is preferably a 3-dibenzofuranyl group and a 4-dibenzofuranyl group, and more preferably the 4-dibenzofuranyl group.

R₁ and R₂ in the general formula (a) or (b) are preferably a hydrogen atom, phenyl group, biphenylyl group, naphthyl group, phenanthrenyl group, or carbazolyl group, and more preferably a hydrogen atom, phenyl group, or biphenyl group.

The arylamine compound represented by the general formula (a) or (b), which is preferably used in the organic EL element of the present disclosure, is preferably used as a constituent material of a hole injection layer, hole transport layer, electron blocking layer, or light emitting layer of the organic EL element, and more preferably used as a constituent material of a hole transport layer or an electron blocking layer.

### [Advantageous Effects]

Compared to conventional hole transport materials, the arylamine compound of the present disclosure has characteristics such as (1) good hole injection properties, (2) high hole mobility, (3) excellent electron blocking ability, (4) high electron resistance, (5) stable existence in a thin film state, (6) excellent heat resistance, etc., and characteristics such as (7) high luminous efficiency, (8) low emission start voltage, (9) low practical driving voltage, (10) long lifespan, etc. are obtained by using the arylamine compound of the present disclosure in an organic EL element.

The arylamine compound of the present disclosure is excellent in hole injection/transport performance, and thin film stability and durability. As a result, the organic EL element having a hole injection layer and/or a hole transport layer manufactured using the same compound as a hole injection material and/or a hole transport material improves luminous efficiency by improving the hole transport efficiency to the light emitting layer and enables the durability of the element to be improved by lowering the driving voltage, thereby making it possible to obtain characteristics of high efficiency, low driving voltage, and long lifespan.

The arylamine compound of the present disclosure has excellent electron blocking ability, has high electron resistance, is stable even in a thin film state, and has the characteristics of confining excitons generated in the light emitting layer. As a result, since the probability of recombining holes and electrons is improved to suppress thermal deactivation in an organic EL element having an electron blocking layer manufactured using the same compound as an electron blocking material, the organic EL element has high luminous efficiency, and the driving voltage is reduced, thereby improving current resistance to improve maximum luminance.

The arylamine compound of the present disclosure has excellent hole transport properties and has a wide band gap. As a result, in an organic EL element having a light emitting layer manufactured using the same compound as a host material, the driving voltage is lowered, and the luminous efficiency is improved by forming the light emitting layer by supporting a fluorescent light emitter, a phosphorescent light emitter, or a delayed fluorescent light emitter, which is called a dopant.

Therefore, the arylamine compound of the present disclosure is useful as a material for the hole injection layer, hole transport layer, electron blocking layer, or light emitting layer of an organic EL element, and can improve the luminous efficiency, driving voltage, and durability of a conventional organic EL element.

In addition, the arylamine compound of the present disclosure can be used not only in organic EL elements but also in the field of electronic devices such as electrophotographic photoreceptors, image sensors, photoelectric conversion elements, and solar cells.

### [Description of Drawings]

FIG. 1 is a drawing showing Compound (1) to Compound (12) as preferred specific examples of an arylamine compound represented by the general formula (a) or (b).
FIG. 2 is a drawing showing Compound (13) to Compound (24) as preferred specific examples of an arylamine compound represented by the general formula (a) or (b).
FIG. 3 is a drawing showing Compound (25) to Compound (36) as preferred specific examples of an arylamine compound represented by the general formula (a) or (b).
FIG. 4 is a drawing showing Compound (37) to Compound (48) as preferred specific examples of an arylamine compound represented by the general formula (a) or (b).
FIG. 5 is a drawing showing Compound (49) to Compound (60) as preferred specific examples of an arylamine compound represented by the general formula (a) or (b).
FIG. 6 is a drawing showing Compound (61) to Compound (72) as preferred specific examples of an arylamine compound represented by the general formula (a) or (b).
FIG. 7 is a drawing showing Compound (73) to Compound (84) as preferred specific examples of an arylamine compound represented by the general formula (a) or (b).
FIG. 8 is a drawing showing Compound (85) to Compound (96) as preferred specific examples of an arylamine compound represented by the general formula (a) or (b).
FIG. 9 is a drawing showing Compound (97) to Compound (111) as preferred specific examples of an arylamine compound represented by the general formula (a) or (b).
FIG. 10 is a drawing showing Compound (112) to Compound (123) as preferred specific examples of an arylamine compound represented by the general formula (a) or (b).
FIG. 11 is a drawing showing Compound (124) to Compound (138) as preferred specific examples of an arylamine compound represented by the general formula (a) or (b).
FIG. 12 is a drawing showing Compound (139) to Compound (150) as preferred specific examples of an arylamine compound represented by the general formula (a) or (b).
FIG. 13 is a drawing showing Compound (151) to Compound (165) as preferred specific examples of an arylamine compound represented by the general formula (a) or (b).
FIG. 14 is a drawing showing Compound (166) to Compound (177) as preferred specific examples of an arylamine compound represented by the general formula (a) or (b).
FIG. 15 is a drawing showing Compound (178) to Compound (190) as preferred specific examples of an arylamine compound represented by the general formula (a) or (b).
FIG. 16 is a drawing showing Compound (191) to Compound (203) as preferred specific examples of an arylamine compound represented by the general formula (a) or (b).
FIG. 17 is a drawing showing Compound (204) to Compound (215) as preferred specific examples of an arylamine compound represented by the general formula (a) or (b).
FIG. 18 is a drawing showing Compound (216) to Compound (220) as preferred specific examples of an arylamine compound represented by the general formula (a) or (b).
FIG. 19 is a drawing showing the organic EL element configurations of Examples 19 to 34 and Comparative Examples 1 and 2.

### [Best mode for carrying out the invention]

Although the arylamine compounds of the present disclosure are novel compounds, these compounds can be synthesized according to methods known per se.

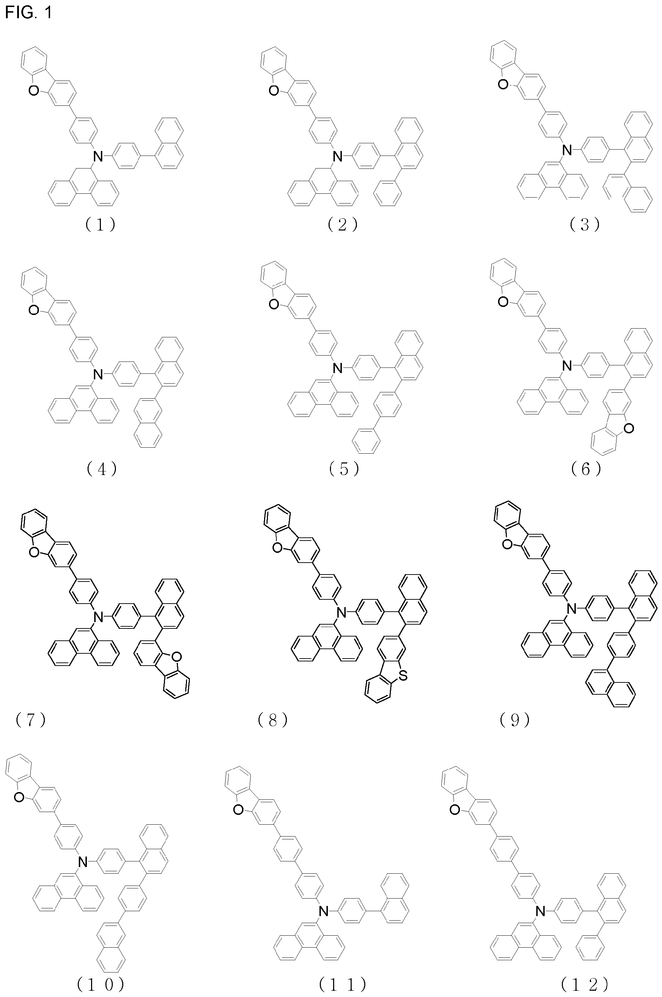

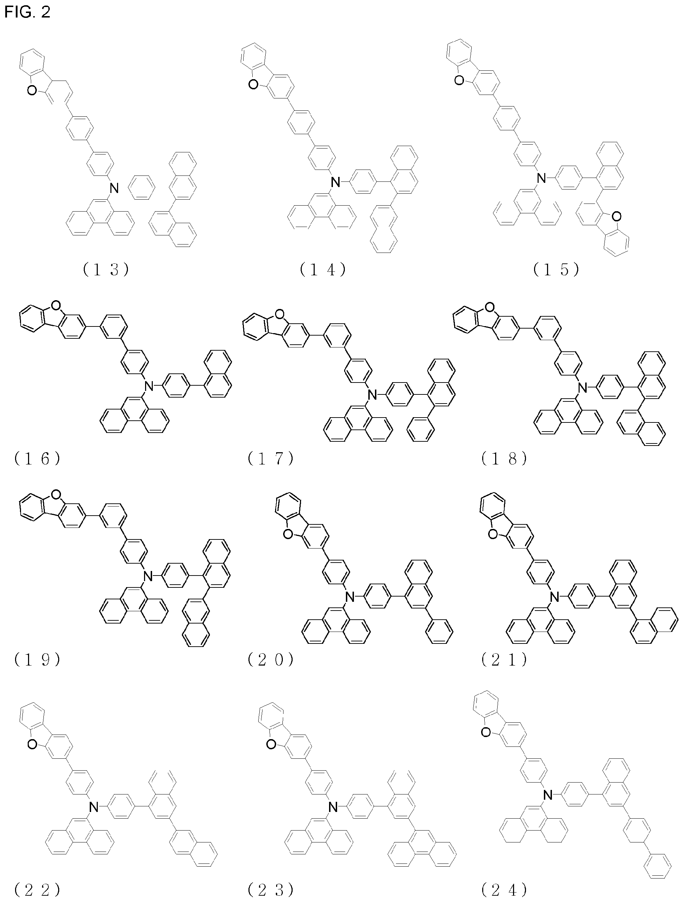

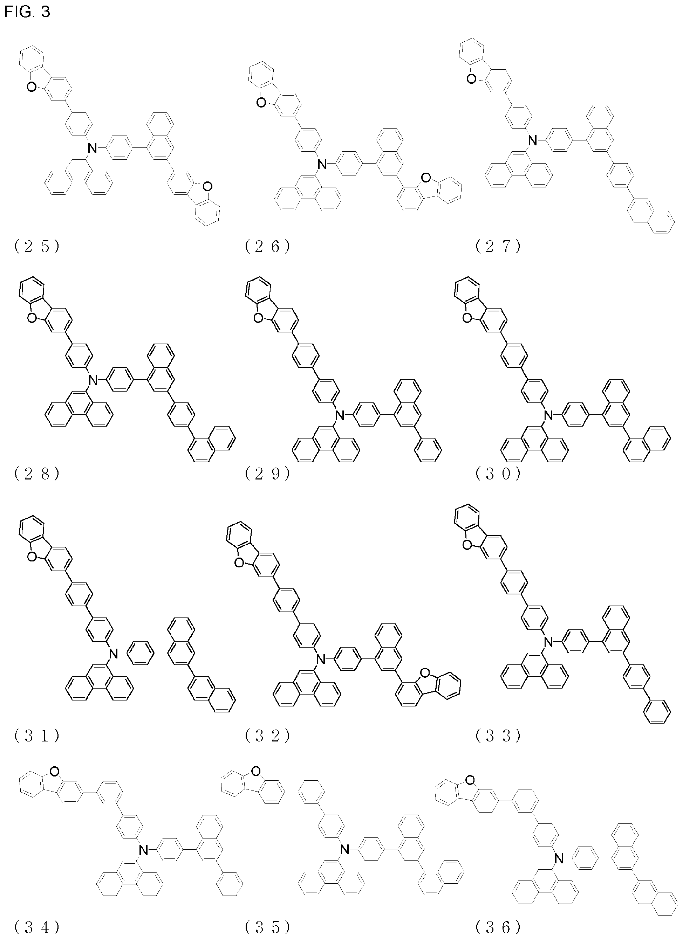

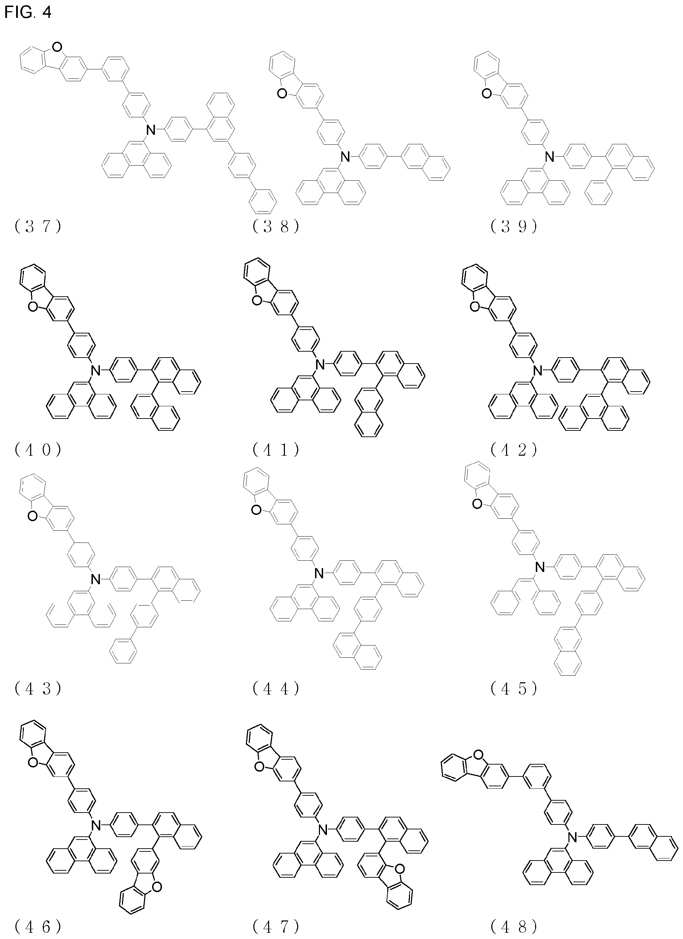

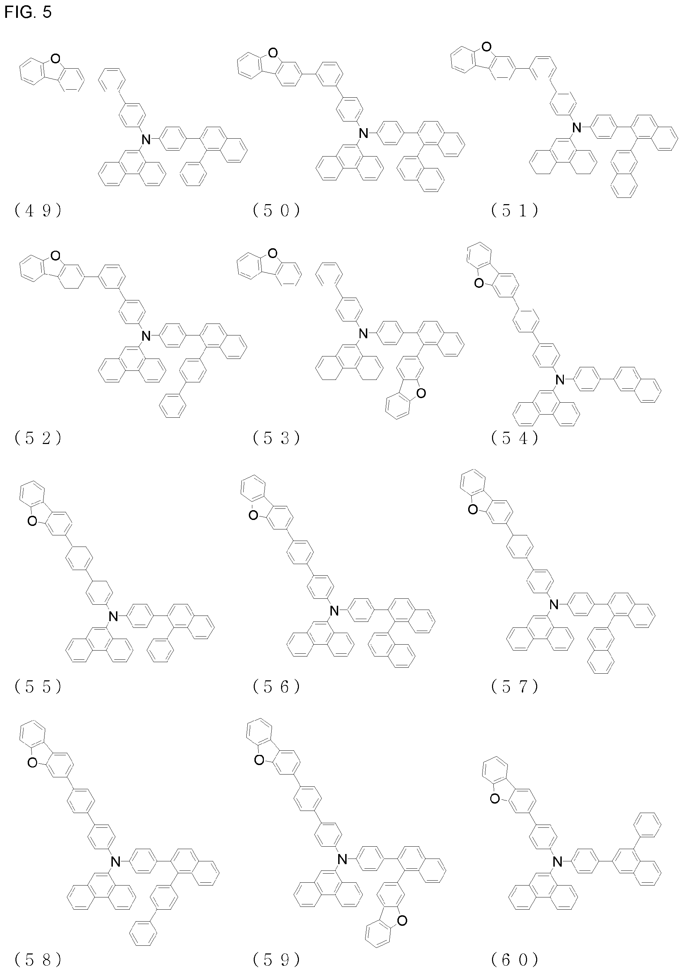

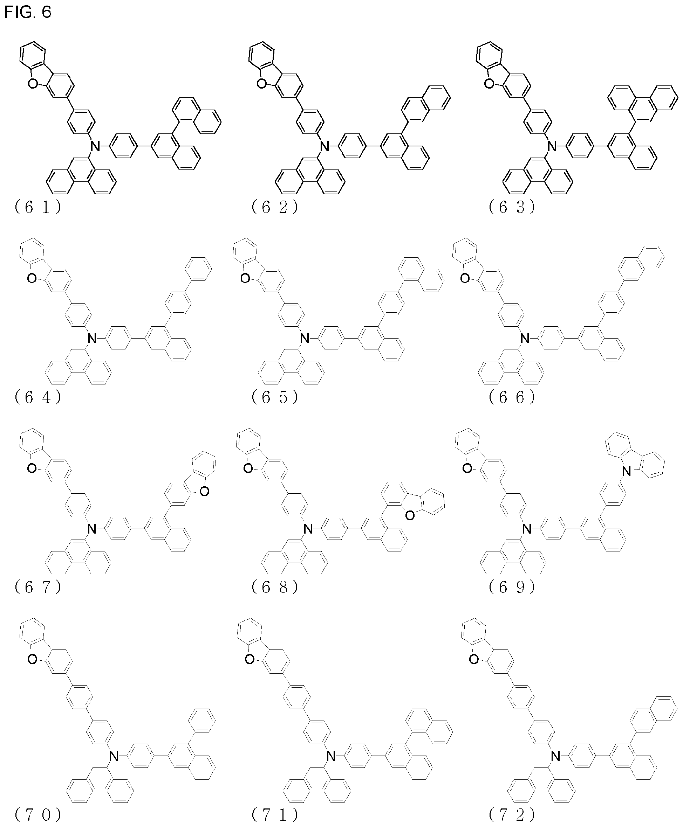

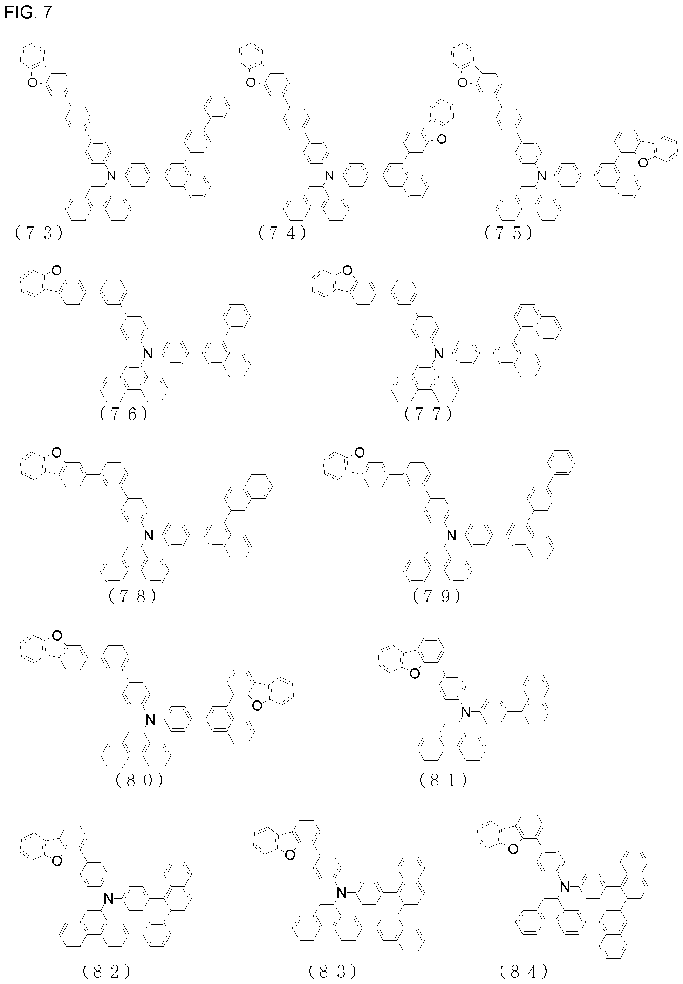

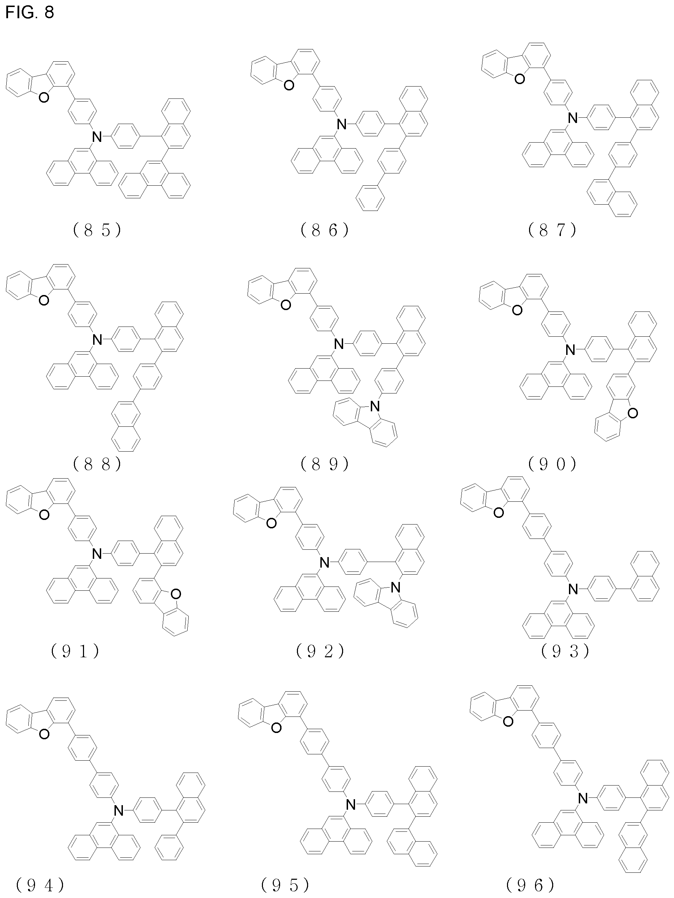

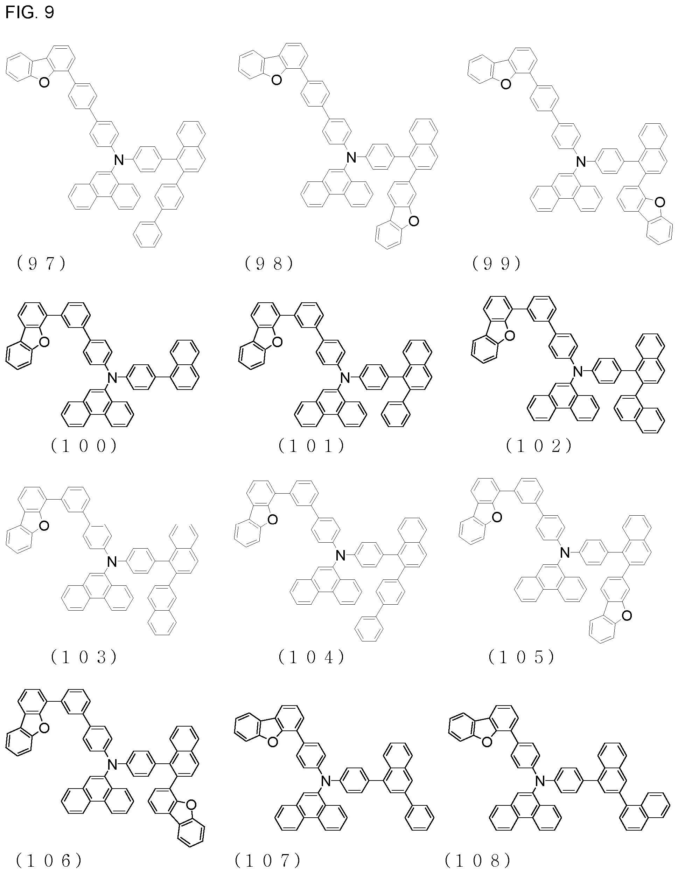

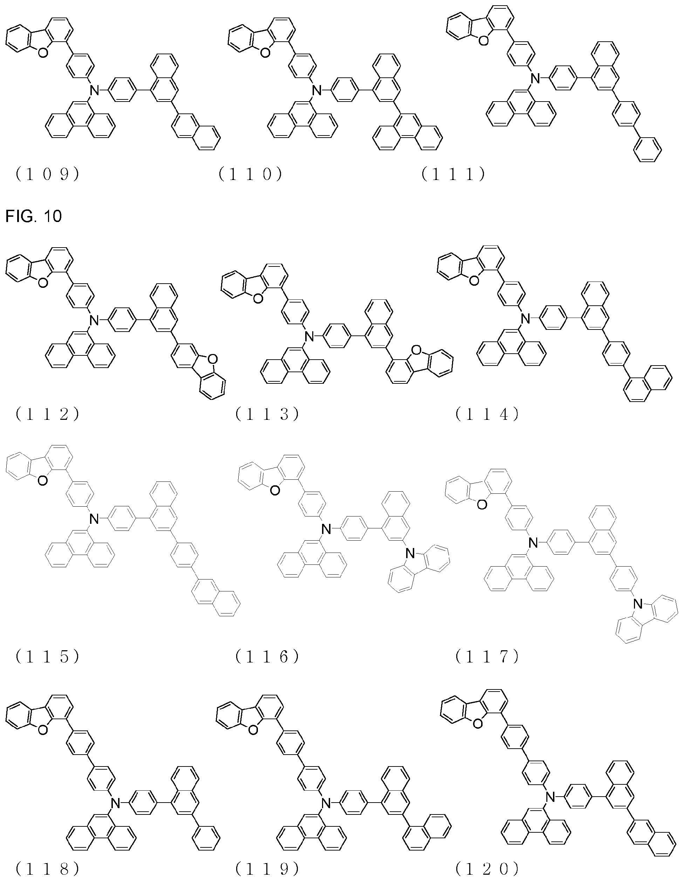

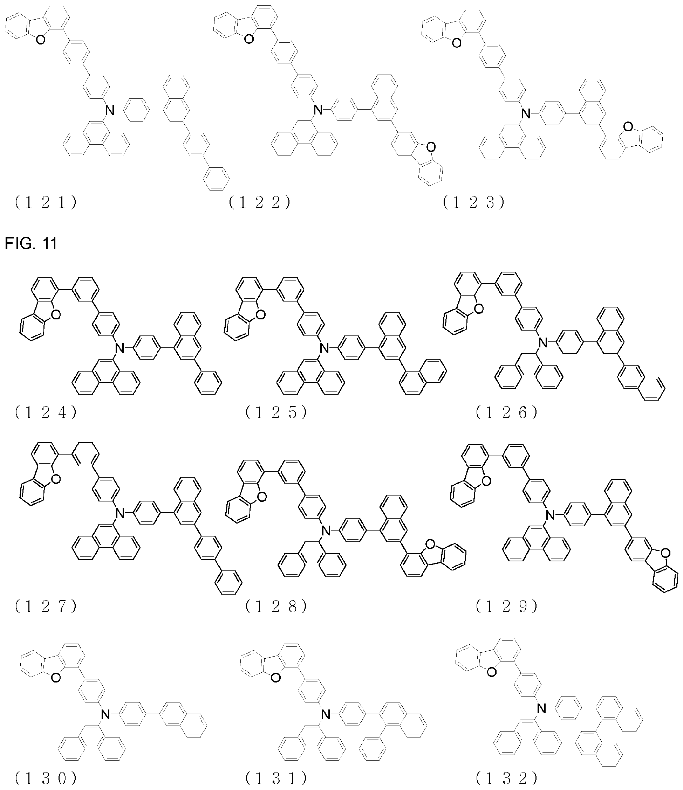

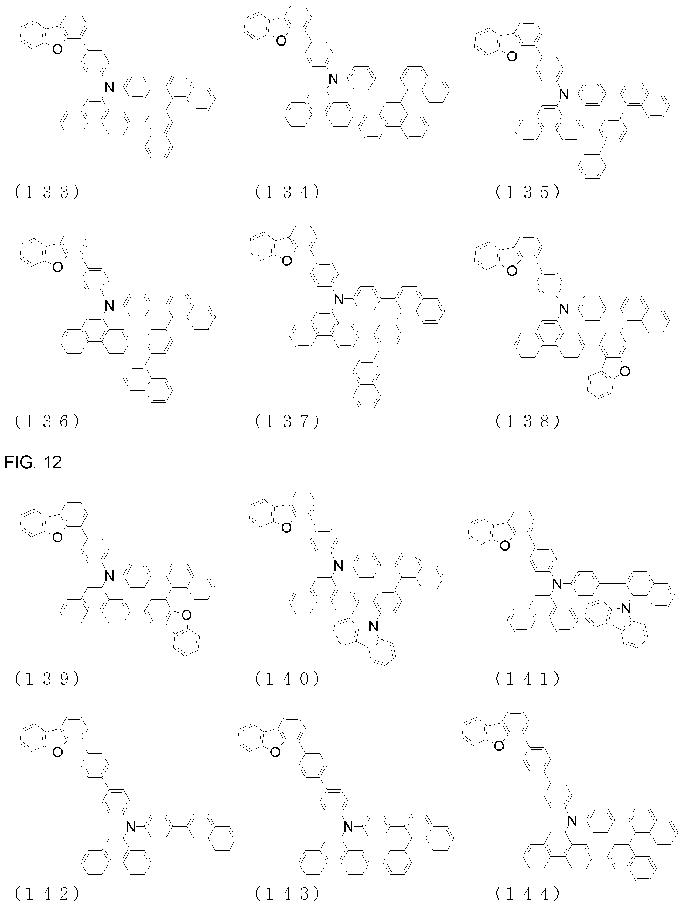

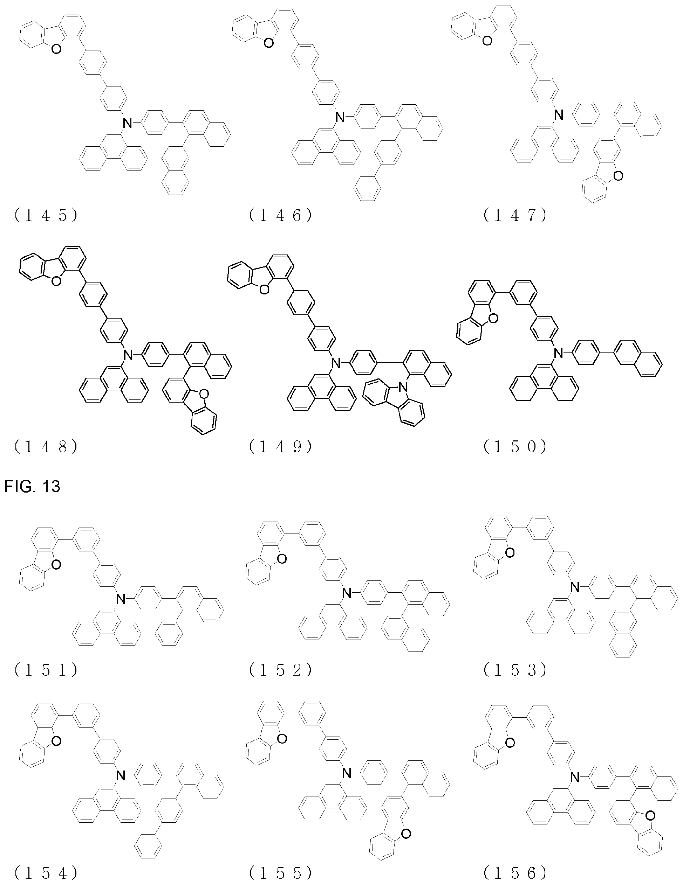

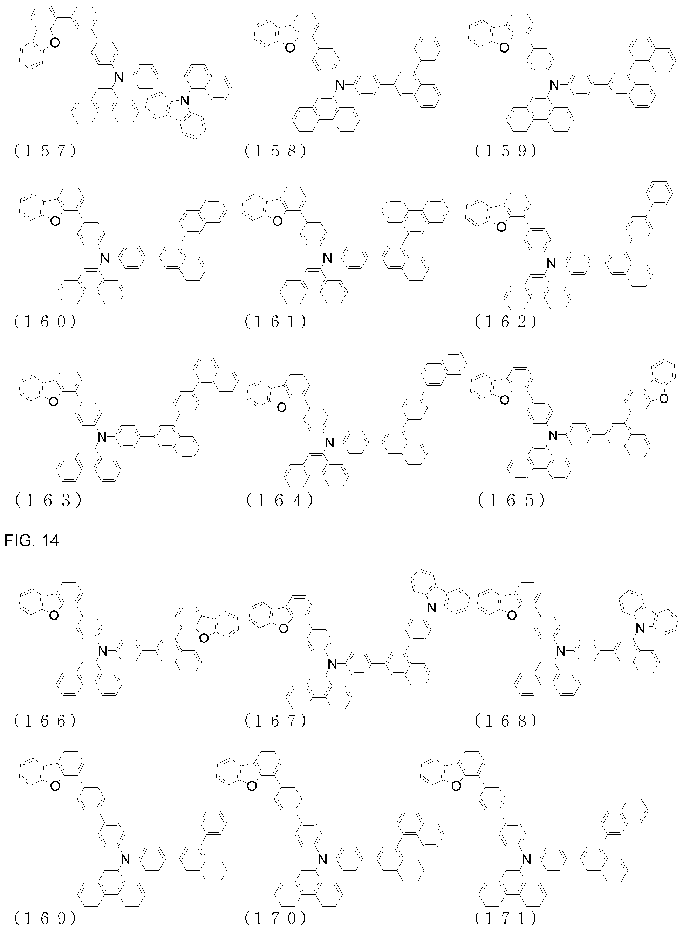

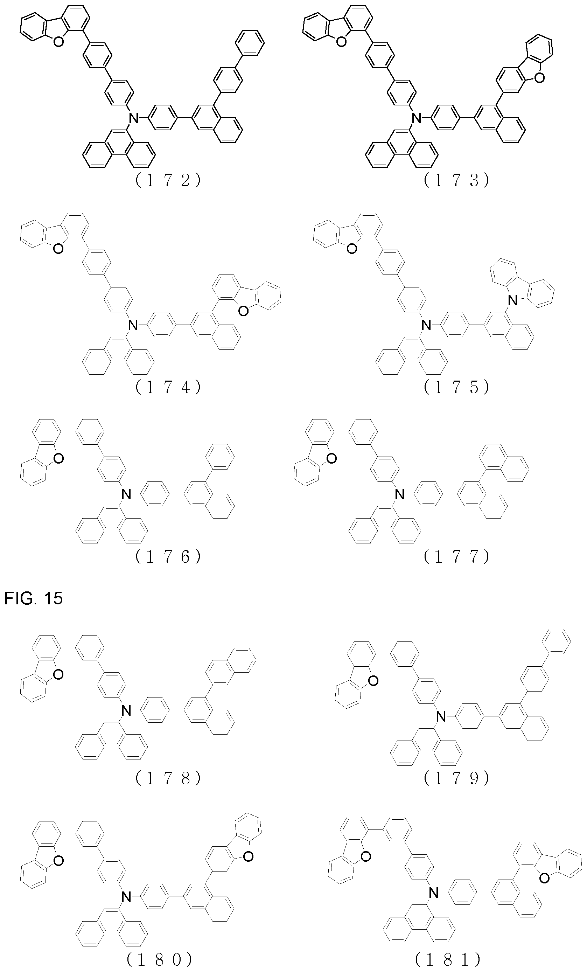

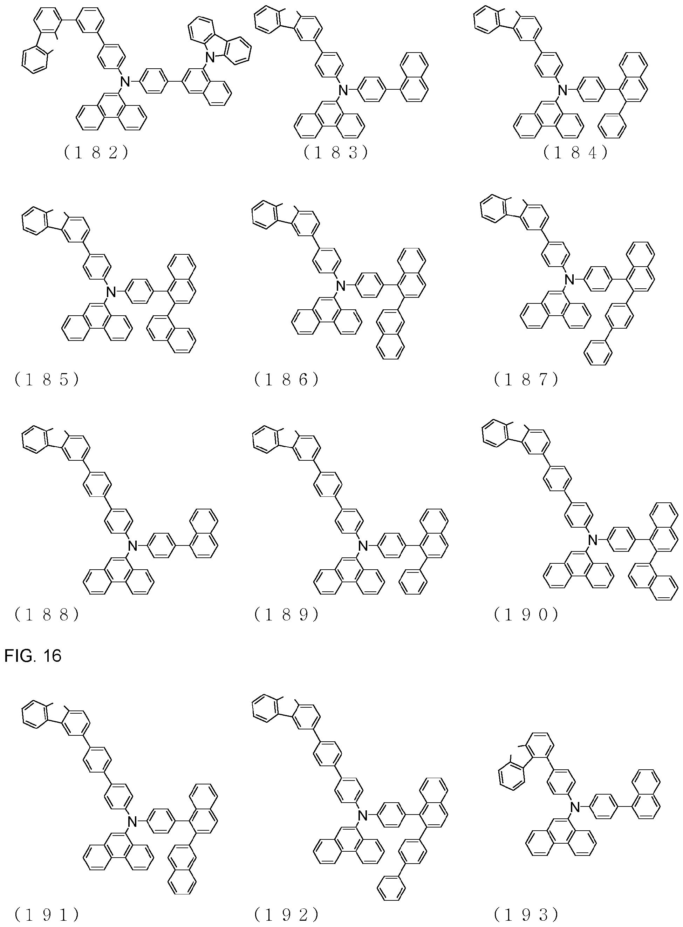

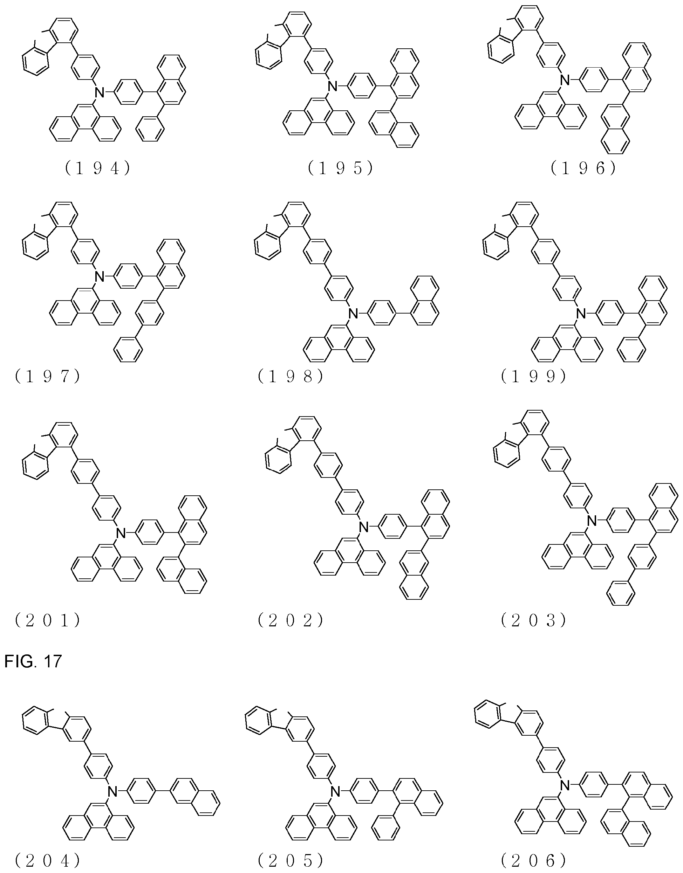

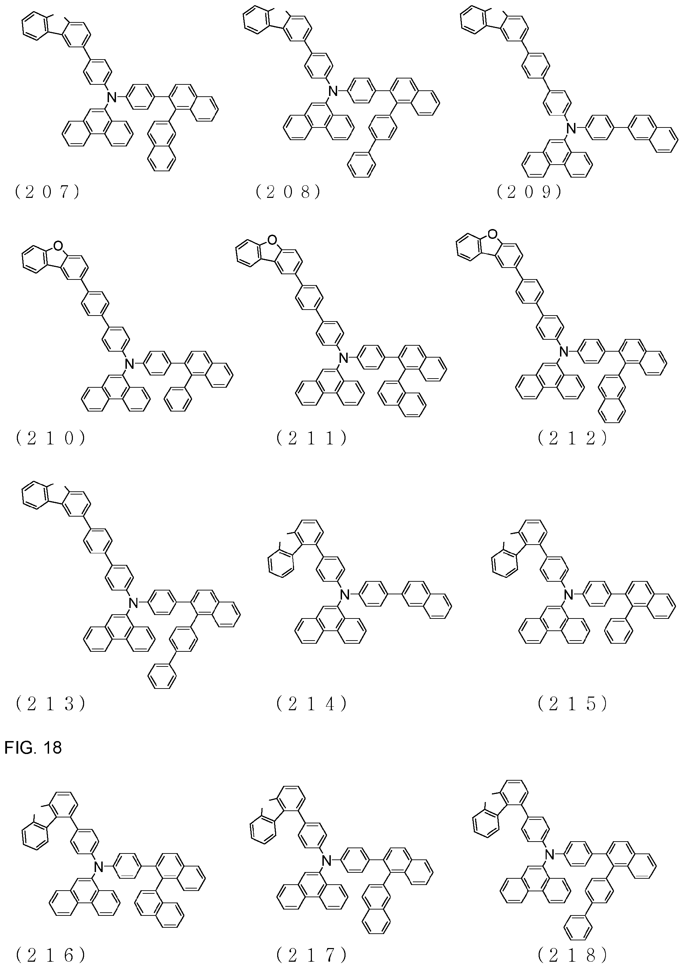

Among the arylamine compounds represented by the general formula (a) or (b), which are preferably used in the organic EL element of the present disclosure, specific examples of preferable compounds are shown in FIGS. 1 to 18, but are not limited to these compounds.

Purification of the arylamine compound represented by the general formula (a) or (b) can be carried out by known methods such as a purification method by column chromatography, an adsorption purification method by silica gel, activated carbon, activated clay, etc., a recrystallization or crystallization method by a solvent, a sublimation purification method, etc. Identification of compounds can be performed by NMR analysis. Physical property values may include melting point, glass transition point (Tg), and work function measurements. The melting point is an indicator of deposition properties, the glass transition point (Tg) is an indicator of the stability of the thin film state, and the work function is an indicator of hole injection properties, hole transport properties, or electron blocking properties.

The melting point and the glass transition point (Tg) can be measured, for example, with a high-sensitivity differential scanning calorimeter (DSC3100SA, manufactured by Bruker AXS) using powder.

The work function can be determined, for example, by manufacturing a 100 nm thin film on an ITO substrate and using an ionization potential measurement device (PYS-202, manufactured by Sumitomo Heavy Industries, Ltd.).

The structure of the organic EL element of the present disclosure may include a structure in which an anode, a hole injection layer, a hole transport layer, a light emitting layer, an electron transport layer, an electron injection layer and a cathode are sequentially formed on a substrate, a structure in which an electron blocking layer is formed between the hole transport layer and the light emitting layer, and a structure in which a hole blocking layer is formed between the light emitting layer and the electron transport layer. In these multilayer structures, it is possible for one organic layer to play the role of several layers, and for example, one organic layer can have a structure that also serves as a hole injection layer and a hole transport layer, or it can have a structure that also serves as an electron injection layer and an electron transport layer. In addition, it is possible to have a configuration in which two or more organic layers with the same function are stacked, and a configuration in which two layers of a hole transport layer are stacked, a configuration in which two layers of a light emitting layer are stacked, and a configuration in which two layers of an electron transport layer are stacked can be used.

As the anode of the organic EL element of the present disclosure, an electrode material with a large work function such as ITO or gold is used. As a material for the hole injection layer of the organic EL element of the present disclosure, a porphyrin compound represented by copper phthalocyanine, a starburst type triphenylamine derivative, an arylamine compound having two or more triphenylamine structures or carbazolyl structures in the molecule, each of which has a structure linked by a divalent group that does not contain a single bond or heteroatom, acceptor heterocyclic compounds such as hexacyanoazatriphenylene, and coating-type polymer materials can be used. These materials can be formed into thin films by known methods such as vapor deposition, spin coating, and inkjet methods.

As a material for the hole injection layer and hole transport layer of the organic EL element of the present disclosure, in addition to the arylamine compound of the present disclosure, benzidine derivatives such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylylbenzidine, 1,1-Bis[(di-4-tolylamino)phenyl]cyclohexane (TAPC), and an arylamine compound having two or more triphenylamine structures or carbazolyl structures in the molecule, each of which has a structure linked by a divalent group that does not contain a single bond or heteroatom can be used. These materials may be formed into a film individually, but may be formed into a film by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. In addition, as a material for the hole injection/transport layer, a coating type polymer material such as poly(3,4-ethylenedioxythiophene) (PEDOT)/poly(styrenesulfonate) (PSS) can be used. These materials can be formed into thin films by known methods such as vapor deposition, spin coating, and inkjet methods.

In addition, in these layers in the hole injection layer or the hole transport layer, those in which trisbromophenylamine hexachloroantimony, radialene derivatives (for example, see Patent Document 6), etc. are P-doped, polymer compounds having the structure of benzidine derivatives such as TPD in their partial structures, etc. can be used with respect to a material commonly used.

As a material for the electron blocking layer of the organic EL element of the present disclosure, in addition to the arylamine compound of the present disclosure, compounds having an electron blocking function, such as compounds having a triphenylsilyl group and a triarylamine structure represented by carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz), and 9-[4-(carbazole)-9-yl)phenyl]-9-[4-(triphenylsilyl)phenyl]-9H-fluorene, can be used. These materials may also serve as materials for the hole transport layer. These materials may be formed into a film individually, but may be formed by mixing multiple types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into a thin film by known methods such as vapor deposition, spin coating, and inkjet methods.

As a material for the light emitting layer of the organic EL element of the present disclosure, in addition to the arylamine compound of the present disclosure, metal complexes of quinolinol derivatives including tris(8-quinolinolato)aluminum (Alqs), and various metal complexes, anthracene derivatives, bis-styrylbenzene derivatives, pyrene derivatives, oxazole derivatives, polyparaphenylenevinylene derivatives, etc. can be used. In addition, the light emitting layer may be composed of a host material and a dopant material, and an anthracene derivative is preferably used as the host material. However, in addition to the above light emitting materials including the arylamine compound of the present disclosure, heterocyclic compounds having an indole ring as a partial structure of a condensed ring, heterocyclic compounds having a carbazole ring as a partial structure of a condensed ring, carbazole derivatives, thiazole derivatives, benzimidazole derivatives, polydialkylfluorene derivatives, etc. may be used. Also, quinacridone, coumarin, rubrene, perylene and their derivatives, benzopyran derivatives, rhodamine derivatives, aminostyryl derivatives, etc. may be used as the dopant material. These materials may be formed into a film individually, but may be formed into a film by mixing a plurality of types, or each of them may be used as a single layer. In addition, it may be a laminated structure of layers formed by forming a film of these materials alone, a laminated structure of layers formed into a film by mixing these materials, or a laminated structure of a layer formed of these materials alone and a layer formed by mixing a plurality of types. These materials may be formed into thin films by known methods such as vapor deposition, spin coating, and inkjet methods.

In addition, it is also possible to use a phosphorescent emitter as a light-emitting material. A phosphorescent emitter of a metal complex such as iridium or platinum may be used as the phosphorescent emitter. For example, the phosphorescent emitter may include a green phosphorescent emitter such as Ir(ppy)₃, a blue phosphorescent emitter such as FIrpic or Fire, a red phosphorescent emitter such as Btp₂Ir(acac), etc. The host material at this time may include, as a host material with hole injection/transport properties, the arylamine compound of the present disclosure in addition to carbazole derivatives such as 4,4'-di(N-carbazolyl)biphenyl (CBP), TCTA, and mCP, and may include, as a host material with electron transport properties, p-bis(triphenylsilyl)benzene (UGH2), 2,2',2"-(1,3,5-phenylene)-tris(1-phenyl-1H-benzimidazole) (TPBI), or the like. A high-performance organic EL element may be manufactured by using such materials.

In order to avoid concentration quenching, doping of the host material of the phosphorescent light-emitting material is preferably carried out by co-deposition in the range of 1 to 30 weight percent with respect to the entire light emitting layer.

In addition, it is also possible to use a material that emits delayed fluorescence, such as CDCB derivatives such as PIC-TRZ, CC2TA, PXZ-TRZ, and 4CzIPN as the light-emitting material (e.g., see Non-Patent Document 3). These materials may be formed into thin films by known methods such as vapor deposition, spin coating, and inkjet methods.

As a material for the hole blocking layer of the organic EL element of the present disclosure, phenanthroline derivatives such as basocuproine (BCP), metal complexes of quinolinol derivatives such as bis(2-methyl-8-quinolinolate)-4-(phenylphenolato)aluminum (BAlq), various rare earth complexes, and compounds having a hole blocking effect, such as oxazole derivatives, triazole derivatives, and triazine derivatives, may be used. These materials may also serve as materials for the electron transport layer. These materials may be formed into a film individually, but may be formed into a film by mixing a plurality of types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

As a material for the electron transport layer of the organic EL element of the present disclosure, metal complexes of quinolinol derivatives including Alqs and BAIq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives, and silole derivatives may be used. These materials may be formed into a film individually, but may be formed into a film by mixing a plurality of types, or each of them may be used as a single layer. In addition, it may be a laminated structure of layers formed by forming a film of these materials alone, a laminated structure of layers formed by mixing these materials, or a laminated structure of a layer formed of these materials alone and a layer formed by mixing a plurality of types. These materials may be formed into thin films by known methods such as vapor deposition, spin coating, and inkjet methods.

As a material for the electron injection layer of the organic EL element of the present disclosure, alkali metal salts such as lithium fluoride and cesium fluoride, alkaline earth metal salts such as magnesium fluoride, metal complexes of quinolinol derivatives such as lithium quinolinol, metal oxides such as aluminum oxide, and metals such as ytterbium (Yb), samarium (Sm), calcium (Ca), strontium (Sr), and cesium (Cs) may be used. The electron injection layer may be omitted depending on the desirable choice of the electron transport layer and cathode.

In addition, in the electron injection layer and the electron transport layer, those in which a metal such as cesium is N-doped with respect to materials commonly used in these layers may be used.

In the cathode of the organic EL element of the present disclosure, a metal with a low work function, such as aluminum, and an alloy with a lower work function, such as a magnesium silver alloy, a magnesium indium alloy, and an aluminum magnesium alloy, are used as electrode materials.

### [Mode for carrying out the invention]

### Example

Hereinafter, embodiments of the present disclosure will be specifically described through Examples, but the present disclosure is not limited to the following Examples as long as the gist is not exceeded.

### [Example 1]

### <Synthesis of 4-(dibenzofuran-3-yl)phenyl-4-(naphthalen-1-yl)phenyl-phenanthren-9-yl-amine (Compound 1)>

18.1 g of 9-bromo-phenanthrene, 18.5 g of 1-(4-aminophenyl)naphthalene, 0.3 g of Tris(dibenzylideneacetone)dipalladium (0), 0.9 g of 2,2'-Bis(diphenylphosphino)-1,1'-binaphthyl, and 8.8 g of sodium tert-butoxide were injected into a reaction vessel, and the mixture was refluxed and stirred under a toluene solvent for 15 hours. After checking the completion of the reaction, the filtrate obtained by filtration was concentrated to obtain a crude product. The obtained crude product was subjected to crystallization and purification using a chlorobenzene/acetone mixed solvent to obtain 24.4 g of 4-(naphthalen-1-yl)phenyl-phenanthren-9-yl-amine (yield: 87.8%).

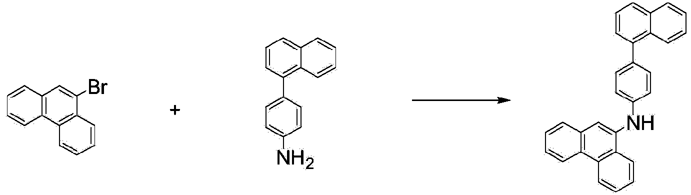

Subsequently, 11.2 g of 4-(naphthalen-1-yl)phenyl-phenanthren-9-yl-amine, 10.1 g of 3-(4-bromophenyl)dibenzofuran, 0.1 g of palladium(II) acetate, 0.2 g of Tri-tert-butylphosphine, 3.5 g of sodium tert-butoxide were injected into the reaction vessel, and the mixture was refluxed and stirred under a toluene solvent for 15 hours. After checking the completion of the reaction, the filtrate obtained by filtration was concentrated to obtain a crude product. The obtained crude product was purified by column chromatography (carrier: silica gel, eluent: dichloromethane/n-heptane) to obtain 11.0 g of a white powder of 4-(dibenzofuran-3-yl)phenyl-4-(naphthalene-1-yl)phenyl-phenanthren-9-yl-amine (Compound 1) (yield: 60.8%).

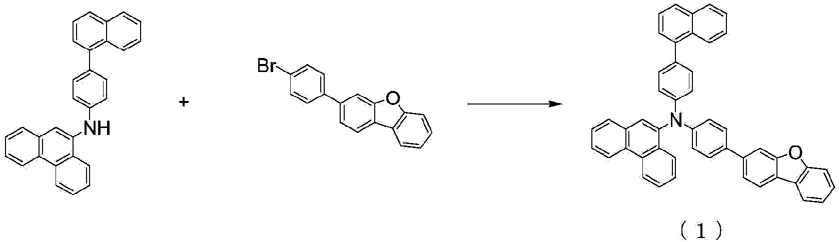

The structure of the obtained white powder was identified using NMR.

The following 31 hydrogen signals were detected by ¹H-NMR (CDCl₃). δ (ppm) = 8. 79 (1H), 8. 74 (1H), 8. 18 (1H), 8. 03 (1 H), 7. 96 (1H), 7. 94 (1H), 7. 8 9 (1H), 7. 84 (2H), 7. 77 (2H), 7. 69 (2H), 7. 60 (2H), 7. 57 (4H), 7. 50 (2 H), 7. 45 (3H), 7. 41-7. 26 (7H).

### [Example 2]

### <Synthesis of 4-(dibenzofuran-3-yl)phenyl-4-(naphthalen-2-yl)phenyl-phenanthren-9-yl-amine (Compound 38)>

47.2 g of 9-bromo-phenanthrene, 48.3 g of 2-(4-aminophenyl)naphthalene, 0.8 g of Tris(dibenzylideneacetone)dipalladium (0), 2.3 g of 2,2'-Bis(diphenylphosphino)-1,1'-binaphthyl, and 26.5 g of sodium tert-butoxide were injected into a reaction vessel, and the mixture was refluxed and stirred under a toluene solvent for 15 hours. After checking the completion of the reaction, the filtrate obtained by filtration was concentrated to obtain a crude product. The obtained crude product was subjected to crystallization and purification using a chlorobenzene/acetone mixed solvent to obtain 51.0 g of 4-(naphthalen-2-yl)phenyl-phenanthren-9-yl-amine (yield: 70.2%).

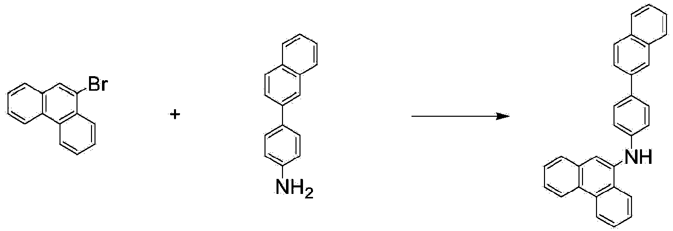

Subsequently, 11.2 g of 4-(naphthalen-2-yl)phenyl-phenanthren-9-yl-amine, 10.1 g of 3-(4-bromophenyl)dibenzofuran, 0.1 g of palladium(II) acetate, 0.2 g of Tri-tert-butylphosphine, 3.5 g of sodium tert-butoxide were injected into the reaction vessel, and the mixture was refluxed and stirred under a toluene solvent for 19 hours. After checking the completion of the reaction, the filtrate obtained by filtration was concentrated to obtain a crude product. The obtained crude product was purified by column chromatography (carrier: silica gel, eluent: dichloromethane/n-heptane) to obtain 11.7 g of a white powder of 4-(dibenzofuran-3-yl)phenyl-4-(naphthalene-2-yl)phenyl-phenanthren-9-yl-amine (Compound 38) (yield: 64.8%).

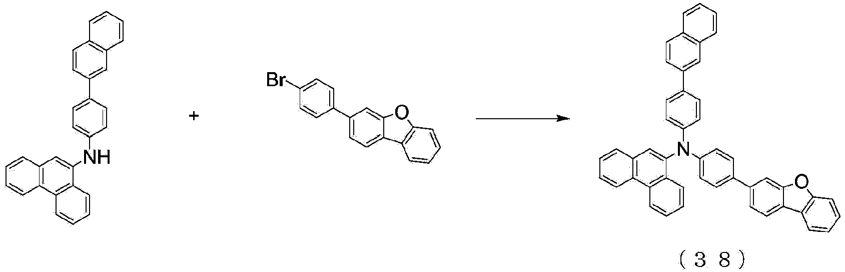

The structure of the obtained white powder was identified using NMR.

The following 31 hydrogen signals were detected by ¹H-NMR (CDCl₃). δ (ppm) =8. 7 9 (1H), 8. 7 4 (1H), 8. 13 (1H), 8. 00 (1 H), 7. 95 (2H), 7. 8 7 (2H), 7. 8 3 (2H), 7. 77 - 7.66 ( 5H), 7.63 - 7. 50 (8H), 7. 4 6 (3H), 7. 34 (1H), 7. 2 7 (4H).

### [Example 3]

### <Synthesis of 4-(dibenzofuran-4-yl)phenyl-4-(naphthalen-1-yl)phenyl-phenanthren-9-yl-amine (Compound 81)>

7.6 g of 4-(naphthalen-1-yl)phenyl-phenanthren-9-yl-amine, 6.6 g of 4-(4-bromophenyl)dibenzofuran, 0.1 g of palladium (II) acetate, 0.2 g of Tri-tert-butylphosphine, and 2.4 g of sodium tert-butoxide were injected into a reaction vessel, and the mixture was refluxed and stirred under a toluene solvent for 14 hours. After checking the completion of the reaction, the filtrate obtained by filtration was concentrated to obtain a crude product. The obtained crude product was purified by column chromatography (carrier: silica gel, eluent: dichloromethane/n-heptane) to obtain 3.9 g of a white powder of 4-(dibenzofuran-4-yl)phenyl-4-(naphthalene-1-yl)phenyl-phenanthren-9-yl-amine (Compound 81) (yield: 31.8%).

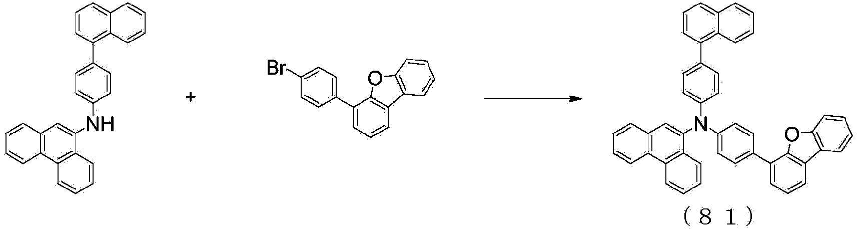

The structure of the obtained white powder was identified using NMR.

The following 31 hydrogen signals were detected by ¹H-NMR (CDCl₃). δ (ppm) = 8. 81 (1H), 8.75 (1H), 8. 22 (1H), 8.04 (1 H), 7. 98 (1H), 7. 93-7. 80 (7H), 7. 70 (2H), 7. 64 ( 1H), 7. 59 (3H), 7. 54-7.41 (6H), 7. 41-7.31 (7H) a

### [Example 4]

### <Synthesis of 4-(dibenzofuran-4-yl)phenyl-4-(naphthalen-2-yl)phenyl-phenanthren-9-yl-amine (Compound 130)>

7.6 g of 4-(naphthalen-2-yl)phenyl-phenanthren-9-yl-amine, 6.6 g of 4-(4-bromophenyl)dibenzofuran, 0.1 g of palladium (II) acetate, 0.2 g of Tri-tert-butylphosphine, and 2.4 g of sodium tert-butoxide were injected into a reaction vessel, and the mixture was refluxed and stirred under a toluene solvent for 14 hours. After checking the completion of the reaction, the filtrate obtained by filtration was concentrated to obtain a crude product. The obtained crude product was purified by column chromatography (carrier: silica gel, eluent: dichloromethane/n-heptane) to obtain 5.5 g of a white powder of 4-(dibenzofuran-4-yl)phenyl-4-(naphthalene-2-yl)phenyl-phenanthren-9-yl-amine (Compound 130) (yield: 44.8%).

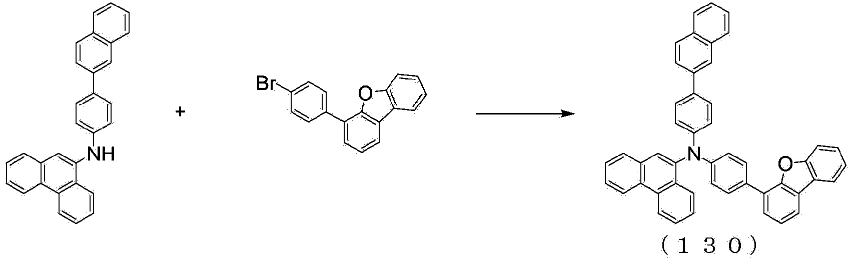

The structure of the obtained white powder was identified using NMR.

The following 31 hydrogen signals were detected by ¹H-NMR (CDCl₃). δ (ppm) = 8. 80 (1H), 8. 75 (1H), 8. 16 (1H), 8. 01 (1 H), 7.98 (1H), 7. 8 8 (3H), 7. 84 (4H) 7. 7 8 (1H), 7. 73 (1H), 7. 6 9 (2H), 7.65 - 7.53 (6H), 7.51-7. 28 ( 9H)∘

### [Example 5]

### <Synthesis of {4'-(dibenzofuran-3-yl)-[1,1'-biphenyl]-4-yl}-4-(naphthalen-1-yl)phenyl-phenanthren-9-yl-amine (Compound 11)>

10.0 g of 4-(naphthalen-1-yl)phenyl-phenanthren-9-yl-amine, 11.1 g of 3-(4'-bromo[1,1'-biphenyl]-4-yl)dibenzofuran, 0.5 g of Tris(dibenzylideneacetone)dipalladium (0), 0.2 g of Tri-tert-butylphosphine, and 4.9 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was subjected to crystallization purification using a mixed solvent of monochlorobenzene/acetone to obtain 12.8 g of a white powder of {4'-(dibenzofuran-3-yl)-[1,1'-biphenyl]-4-yl}-4-(naphthalen-1-yl)phenyl-phenanthren-9-yl-amine (Compound 11) (yield: 71.1%).

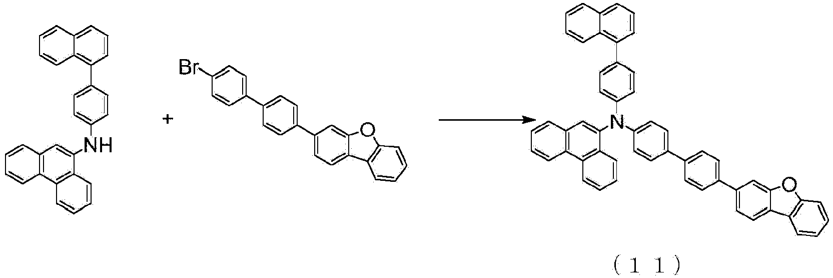

The structure of the obtained white powder was identified using NMR.

The following 35 hydrogen signals were detected by ¹H-NMR (CDCl₃). δ (ppm) = 8.83-8.76 (2H), 8. 21 (1H), 8.07-7.99 (3H), 7.93-7. 8 8 (4H), 7.82(1H), 7. 7 8 - 7 . 4 6 (17 H), 7.43 - 7. 37 (3H), 7. 32 (4H)∘

### [Example 6]

### <Synthesis of {4'-(dibenzofuran-3-yl)-[1,1'-biphenyl]-4-yl}-4-(naphthalen-2-yl)phenyl-phenanthren-9-yl-amine (Compound 54)>

8.0 g of 4-(naphthalen-2-yl)phenyl-phenanthren-9-yl-amine, 8.9 g of 3-(4'-bromo[1,1'-biphenyl]-4-yl)dibenzofuran, 0.4 g of Tris(dibenzylideneacetone)dipalladium (0), 0.2 g of Tri-tert-butylphosphine, and 3.9 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was purified by column chromatography (carrier: silica gel, eluent: dichloromethane/n-heptane) to obtain 7.8 g of a light yellow powder of {4'-(dibenzofuran-3-yl)-[1,1'-biphenyl]-4-yl}-4-(naphthalen-2-yl)phenyl-phenanthren-9-yl-amine (Compound 54) (yield: 54.2%).

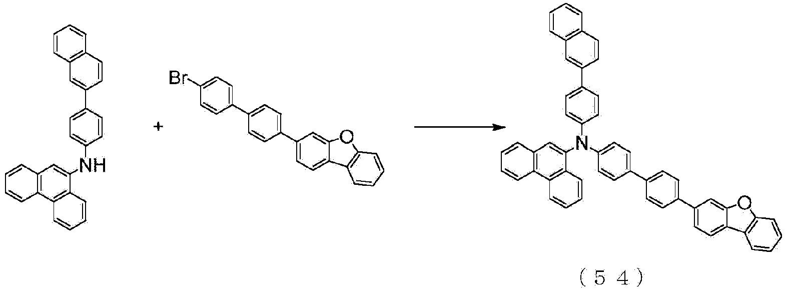

The structure of the obtained light yellow powder was identified using NMR.

The following 35 hydrogen signals were detected by ¹H-NMR (CDCl₃). δ (ppm) = 8.83 - 8. 76 (2H), 8.16 (1H), 8.05-7.99 (3H), 7.92-7.85 (5H), 7.78 - 8.56 (16H), 7.49 (3 H), 7.39 (1H), 7.31-7.27 (4H)∘

### [Example 7]

### <Synthesis of {4'-(dibenzofuran-4-yl)-[1,1'-biphenyl]-4-yl}-4-(naphthalen-1-yl)phenyl-phenanthren-9-yl-amine (Compound 93)>

12.0 g of (4'-bromo-[1,1'-biphenyl]-4-yl)-4-(naphthalen-1-yl)phenyl-phenanthren-9-yl-amine, 4.9 g of 4-dibenzofuranylboronic acid, 0.5 g of Tetrakis(triphenylphosphine)palladium (0), and 5.3 g of potassium carbonate were injected into a reaction vessel, and the mixture was refluxed and stirred overnight under a toluene/EtOH/H₂O mixed solvent. After checking the completion of the reaction, toluene/H₂O was added to the cooled system, and the organic layer was taken out and concentrated through extraction and liquid separation operations to obtain a crude product. The obtained crude product was purified by column chromatography (carrier: silica gel, eluent: dichloromethane/n-heptane) to obtain 4.1 g of a white powder of {4'-(dibenzofuran-4-yl)-[1,1 '-biphenyl]-4-yl}-4-(naphthalen-1 -yl)phenyl-phenanthren-9-yl-amine (Compound 93) (yield: 29.3%).

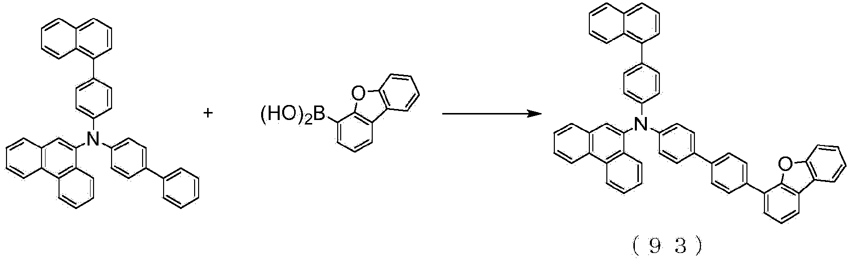

The structure of the obtained white powder was identified using NMR.

The following 35 hydrogen signals were detected by ¹H-NMR (CDCl₃). δ (ppm) = 8.84 - 8. 7 7 (2H), 8. 23 (1H), 8.07 (1H), 8 . 04-8.01 (3H), 7. 97 (1H), 7. 94 - 7.82 (4H), 7.78 - 7. 60(10H), 7.56-7. 45 (6H), 7. 42 - 7.38 (3H), 7 .33 (4H)∘

### [Example 8]

### <Synthesis of {4'-(dibenzofuran-4-yl)-[1,1'-biphenyl]-4-yl}-4-(naphthalen-2-yl)phenyl-phenanthren-9-yl-amine (Compound 142)>

12.0 g of (4'-bromo-[1,1'-biphenyl]-4-yl)-4-(naphthalen-2-yl)phenyl-phenanthren-9-yl-amine, 4.9 g of 4-dibenzofuranylboronic acid, 0.5 g of Tetrakis(triphenylphosphine)palladium (0), and 5.3 g of potassium carbonate were injected into a reaction vessel, and the mixture was refluxed and stirred overnight under a toluene/EtOH/H₂O mixed solvent. After checking the completion of the reaction, toluene/H₂O was added to the cooled system, and the organic layer was taken out and concentrated through extraction and liquid separation operations to obtain a crude product. The obtained crude product was purified by column chromatography (carrier: silica gel, eluent: dichloromethane/n-heptane) to obtain 8.7 g of a white powder of {4'-(dibenzofuran-4-yl)-[1,1'-biphenyl]-4-yl}-4-(naphthalen-2-yl)phenyl-phenanthren-9-yl-amine (Compound 142) (yield: 63.5%).

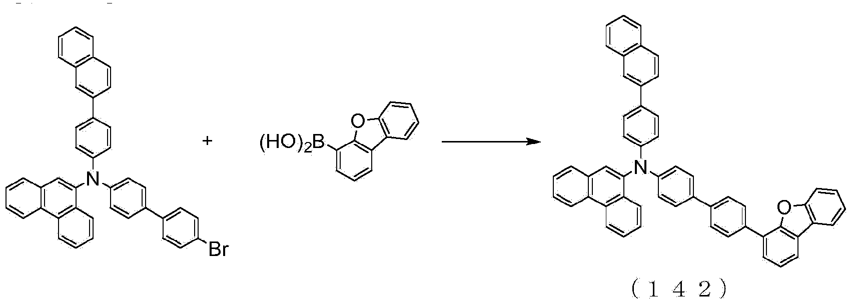

The structure of the obtained white powder was identified using NMR.

The following 35 hydrogen signals were detected by ¹H-NMR (DMSO-d₆). δ (ppm) = 8.99 (1H), 8.92 (1H), 8.22-8.15 (3H), 8 .08 - 7.91 (9H), 7.83-7.61 (12H), 7.57 - 7.42 (5 H), 7.20 (4H)∘

### [Example 9]

### <Synthesis of 4-(dibenzofuran-2-yl)phenyl-4-(naphthalen-1-yl)phenyl-phenanthren-9-yl-amine (Compound 183)>

10.0 g of 4-(dibenzofuran-2-yl)phenyl-phenanthren-9-yl-amine, 7.2 g of 1-(4-bromophenyl)naphthalene, 0.4 g of Tris(dibenzylideneacetone)dipalladium (0), 0.2 g of Tri-tert-butylphosphine, and 3.5 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was purified by column chromatography (carrier: silica gel, eluent: dichloromethane/n-heptane) to obtain 9.1 g of a white powder of 4-(dibenzofuran-2-yl)phenyl-4-(naphthalene-1-yl)phenyl-phenanthren-9-yl-amine (Compound 183) (yield: 62.3%).

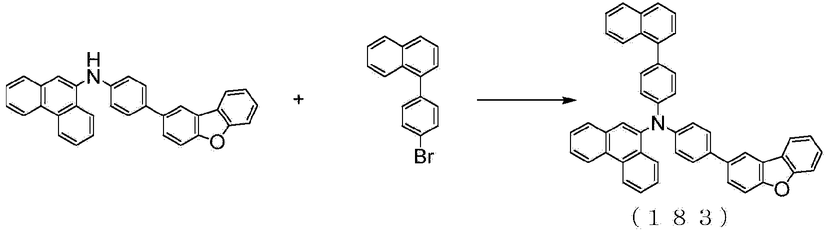

The structure of the obtained white powder was identified using NMR.

The following 31 hydrogen signals were detected by ¹H-NMR (DMSO-d₆). δ (ppm) = 8. 99 (1H), 8. 92 (1H), 8. 41(1H), 8. 20 (1 H), 8. 14 (1H), 8.05 (1H), 8.00- 7.91(4H), 7.76 - 7.64 (9H), 7.57 - 7.52 (4H), 7.45-7.41 (4H), 7.2 7 - 7.22 (4H)∘

### [Example 10]

### <Synthesis of {4'-(dibenzofuran-2-yl)-[1,1'-biphenyl]-4-yl}-4-(naphthalen-1-yl)phenyl-phenanthren-9-yl-amine (Compound 188)>

13.0 g of (4'-bromo-[1,1'-biphenyl]-4-yl)-4-(naphthalen-1-yl)phenyl-phenanthren-9-yl-amine, 5.3 g of 2-dibenzofuranylboronic acid, 0.5 g of Tetrakis(triphenylphosphine)palladium (0), and 5.7 g of potassium carbonate were injected into a reaction vessel, and the mixture was refluxed and stirred overnight under a toluene/EtOH/H₂O mixed solvent. After checking the completion of the reaction, toluene/H₂O was added to the cooled system, and the organic layer was taken out and concentrated through extraction and liquid separation operations to obtain a crude product. The obtained crude product was purified by column chromatography (carrier: silica gel, eluent: dichloromethane/n-heptane) to obtain 9.7 g of a white powder of {4'-(dibenzofuran-2-yl)-[1,1 '-biphenyl]-4-yl}-4-(naphthalen-1 -yl)phenyl-phenanthren-9-yl-amine (Compound 188) (yield: 65.5%).

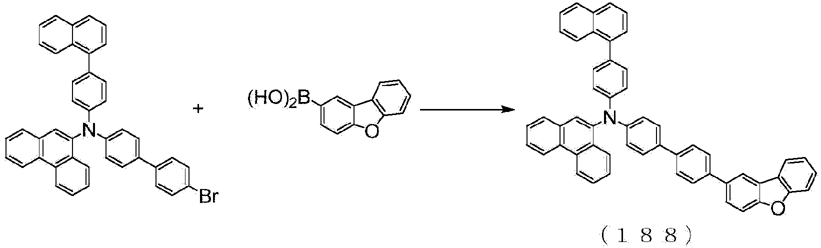

The structure of the obtained white powder was identified using NMR.

The following 35 hydrogen signals were detected by ¹H-NMR (CDCl₃). δ (ppm) = 8.83 - 8.77 (2H), 8.23 - 8.21 (2H), 8. 05 (2H), 7.93 - 7.85 (3H), 7.82 (1H), 7.77 - 7.70 (7H ), 7.67 - 7. 5 8 (6H), 7.56 - 7.46(5H), 7.42 - 7.38( 3 H), 7.32(4H)∘

### [Example 11]

### <Synthesis of 4-(dibenzofuran-1-yl)phenyl-4-(naphthalen-1-yl)phenyl-phenanthren-9-yl-amine (Compound 193)>

8.0 g of 4-(dibenzofuran-1-yl)phenyl-phenanthren-9-yl-amine, 5.7 g of 1-(4-bromophenyl)naphthalene, 0.3 g of Tris(dibenzylideneacetone)dipalladium (0), 0.2 g of Tri-tert-butylphosphine, and 3.5 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was purified by column chromatography (carrier: silica gel, eluent: dichloromethane/n-heptane) to obtain 8.4 g of a yellow powder of 4-(dibenzofuran-1-yl)phenyl-4-(naphthalene-1-yl)phenyl-phenanthren-9-yl-amine (Compound 193) (yield: 71.7%).

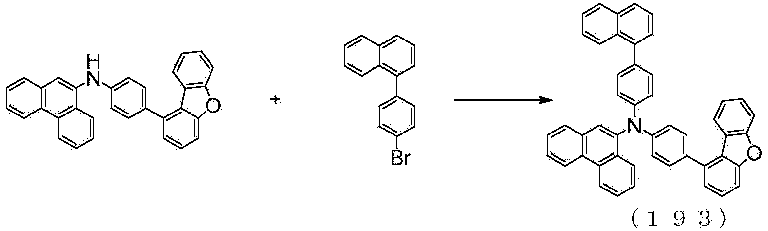

The structure of the obtained yellow powder was identified using NMR.

The following 31 hydrogen signals were detected by ¹H-NMR (DMSO-d₆). δ (ppm) = 9.00 (1H), 8.93 (1H), 8.18 (1H), 8.09 - 8 .05 (2H), 7.99 (1H), 7.93 (2H), 7. 81 - 7.68 (6H), 7.59 - 7.44 (11H), 7.35 - 7.22 (6H)∘

### [Example 12]

### <Synthesis of {4'-(dibenzofuran-1-yl)-[1,1'-biphenyl]-4-yl}-4-(naphthalen-1-yl)phenyl-phenanthren-9-yl-amine (Compound 198)>

12.0 g of (4'-bromo-[1,1'-biphenyl]-4-yl)-4-(naphthalen-1-yl)phenyl-phenanthren-9-yl-amine, 6.2 g of 2-(dibenzofuran-1-yl)-4,4,5,5-tetramethyl-1,3,2-dioxaborolane, 0.5 g of Tetrakis(triphenylphosphine)palladium (0), and 5.3 g of potassium carbonate were injected into a reaction vessel, and the mixture was refluxed and stirred overnight under a toluene/EtOH/H₂O mixed solvent. After checking the completion of the reaction, toluene/H₂O was added to the cooled system, and the organic layer was taken out and concentrated through extraction and liquid separation operations to obtain a crude product. The obtained crude product was purified by column chromatography (carrier: silica gel, eluent: dichloromethane/n-heptane) to obtain 8.5 g of a white powder of {4'-(dibenzofuran-1-yl)-[1,1'-biphenyl]-4-yl}-4-(naphthalen-1-yl)phenyl-phenanthren-9-yl-amine (Compound 198) (yield: 62.0%).

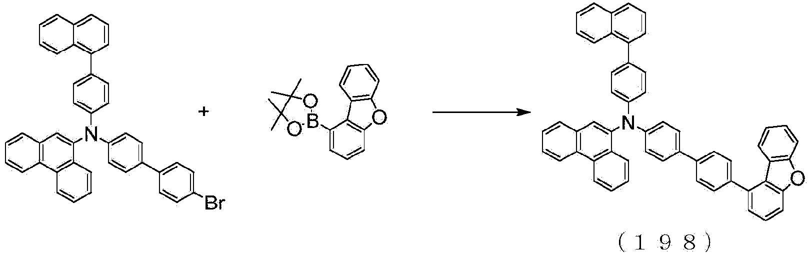

The structure of the obtained white powder was identified using NMR.

The following 35 hydrogen signals were detected by ¹H-NMR (CDCl₃). δ (ppm) = 8. 84 - 8.77 (2H), 8.22 (1H), 8.07 (1H), 7 .94 - 7.85 (3H), 7.82 (1H), 7.79 - 7.76 (2H), 7.72 (4H), 7.67 - 7.59 (7H), 7.57 - 7.41 (8H), 7.36 - 7. 31 (5II), 7.16 (1II)∘

### [Example 13]

### <Synthesis of 4-(dibenzofuran-2-yl)phenyl-4-(naphthalen-2-yl)phenyl-phenanthren-9-yl-amine (Compound 204)>

11.4 g of 4-(dibenzofuran-2-yl)phenyl-phenanthren-9-yl-amine, 8.2 g of 2-(4-bromophenyl)naphthalene, 0.5 g of Tris(dibenzylideneacetone)dipalladium (0), 0.2 g of Tri-tert-butylphosphine, and 5.1 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was purified by column chromatography (carrier: silica gel, eluent: dichloromethane/n-heptane) to obtain 12.5 g of a white powder of 4-(dibenzofuran-2-yl)phenyl-4-(naphthalene-2-yl)phenyl-phenanthren-9-yl-amine (Compound 204) (yield: 74.8%).

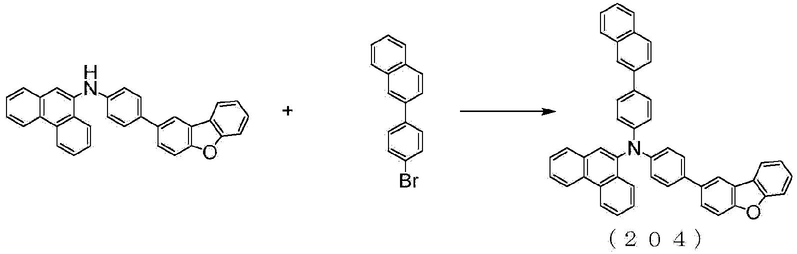

The structure of the obtained white powder was identified using NMR.

The following 31 hydrogen signals were detected by ¹H-NMR (DMSO-d₆). δ (ppm) = 8.98 (1H), 8.91 (1H), 8. 42 (1H), 8.21 - 8 . 16 (2H), 8.08 - 8.01 (2H), 7.98 - 7. 90 (4H), 7. 8 3 - 7.61 (12H), 7.56 - 7.4 7 (3H), 7. 42 (1H), 7. 20 (4 H)∘

### [Example 14]

### <Synthesis of {4'-(dibenzofuran-2-yl)-[1,1'-biphenyl]-4-yl}-4-(naphthalen-2-yl)phenyl-phenanthren-9-yl-amine (Compound 209)>

13.0 g of (4'-bromo-[1,1'-biphenyl]-4-yl)-4-(naphthalen-2-yl)phenyl-phenanthren-9-yl-amine, 5.3 g of 2-dibenzofuranylboronic acid, 0.5 g of Tetrakis(triphenylphosphine)palladium (0), and 5.7 g of potassium carbonate were injected into a reaction vessel, and the mixture was refluxed and stirred overnight under a toluene/EtOH/H₂O mixed solvent. After checking the completion of the reaction, the precipitated solid was filtered under room temperature to obtain a crude product. The obtained crude product was subjected to crystallization and purification using a toluene/acetone mixed solvent to obtain 8.4 g of a white powder of {4'-(dibenzofuran-2-yl)-[1,1'-biphenyl]-4-yl}-4-(naphthalen-2-yl)phenyl-phenanthren-9-yl-amine (Compound 209) (yield: 56.7%).

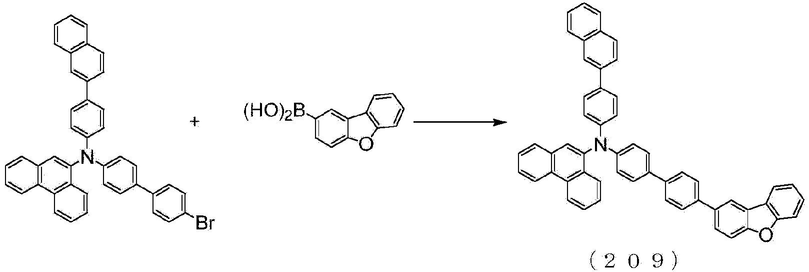

The structure of the obtained white powder was identified using NMR.

The following 35 hydrogen signals were detected by ¹H-NMR (DMSO-d₆). δ (ppm) = 8.98 (1H), 8.92 (1H), 8.53(1H), 8. 25 (1 H), 8.15 (1H). 8.06 (1H), 8.01 (1H), 7.97 - 7. 90 ( 4H), 7.87 - 7.72 (12H), 7.69 - 7.60 (4H), 7.57 - 7. 42 (4H), 7.21 - 7.17 (4H)∘

### [Example 15]

### <Synthesis of 4-(dibenzofuran-1-yl)phenyl-4-(naphthalen-2-yl)phenyl-phenanthren-9-yl-amine (Compound 214)>

9.4 g of 4-(dibenzofuran-1-yl)phenyl-phenanthren-9-yl-amine, 6.7 g of 2-(4-bromophenyl)naphthalene, 0.4 g of Tris(dibenzylideneacetone)dipalladium (0), 0.2 g of Tri-tert-butylphosphine, and 4.2 g of sodium tert-butoxide were injected into a reaction vessel, and the mixture was refluxed and stirred overnight under a toluene solvent. After checking the completion of the reaction, the filtrate obtained by filtration was concentrated to obtain a crude product. The obtained crude product was purified by column chromatography (carrier: silica gel, eluent: dichloromethane/n-heptane) to obtain 8.2 g of a white powder of 4-(dibenzofuran-1-yl)phenyl-4-(naphthalene-1-yl)phenyl-phenanthren-9-yl-amine (Compound 214) (yield: 59.4%).

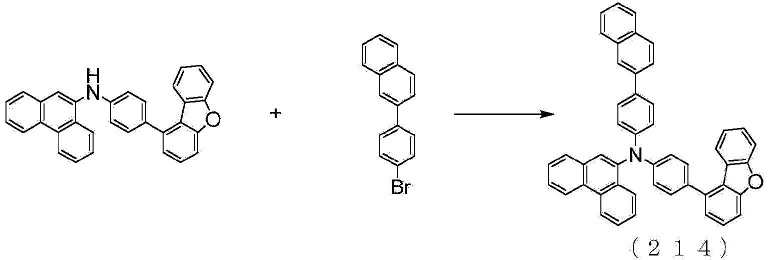

The structure of the obtained white powder was identified using NMR.

The following 31 hydrogen signals were detected by ¹H-NMR (DMSO-d₆). δ (ppm) = 8. 9 9 (1H), 8. 92 (1H), 8.17 (1H), 8.12 (1 H), 8.04 (1H), 7.97 - 7.90 (4H), 7.83 - 7.65 (9H), 7.60 - 7.49 (7II), 7.31 - 7.24 (6II)∘

### [Example 16]

### <Synthesis of {4'-(dibenzofuran-1-yl)-[1,1'-biphenyl]-4-yl}-4-(naphthalen-2-yl)phenyl-phenanthren-9-yl-amine (Compound 219)>

12.0 g of (4'-bromo-[1,1'-biphenyl]-4-yl)-4-(naphthalen-2-yl)phenyl-phenanthren-9-yl-amine, 6.2 g of 2-(dibenzofuran-1-yl)-4,4,5,5-tetramethyl-1,3,2-dioxaborolane, 0.5 g of Tetrakis(triphenylphosphine)palladium (0), and 5.3 g of potassium carbonate were injected into a reaction vessel, and the mixture was refluxed and stirred overnight under a toluene/EtOH/H₂O mixed solvent. After checking the completion of the reaction, toluene/H₂O was added to the cooled system, and the organic layer was taken out and concentrated through extraction and liquid separation operations to obtain a crude product. The obtained crude product was purified by column chromatography (carrier: silica gel, eluent: dichloromethane/n-heptane) to obtain 10.2 g of a white powder of {4'-(dibenzofuran-1-yl)-[1,1'-biphenyl]-4-yl}-4-(naphthalen-2-yl)phenyl-phenanthren-9-yl-amine (Compound 219) (yield: 74.4%).

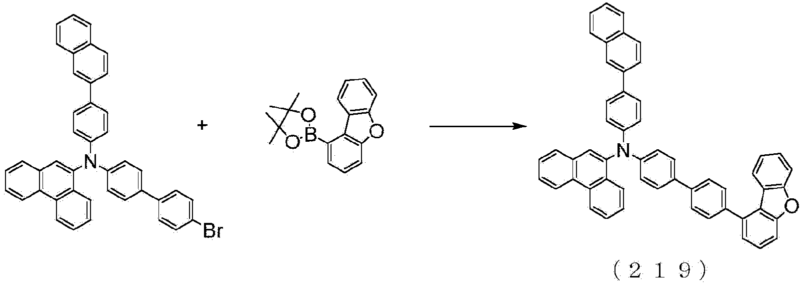

The structure of the obtained white powder was identified using NMR.

The following 35 hydrogen signals were detected by ¹H-NMR (CDCl₃). δ (ppm) = 8.83 - 8.77 (211), 8.17 (1II), 8.04 (1II), 7 . 9 3 - 7 . 8 5 (4H), 7. 79 - 7.75 (8H), 7.6 6 - 7 . 42 (13 H), 7.32 - 7. 30 (5H), 7.17 (1H)∘

### [Example 17]

For the arylamine compounds represented by the general formula (a) or (b), the melting points and glass transition points were measured using a high-sensitivity differential scanning calorimeter (DSC3100SA manufactured by Bruker AXS). The results are shown in Table 1.

**[Table 1]**

| | Melting point | Glass transition point |
|---|---|---|
| Compound (1) | - | 129°C |
| Compound (38) | - | 127°C |
| Compound (81) | - | 131°C |
| Compound (130) | - | 130°C |
| Compound (11) | 280°C | 139°C |
| Compound (54) | - | 138°C |
| Compound (93) | - | 140°C |
| Compound (142) | - | 139°C |
| Compound (183) | - | 128°C |
| Compound (188) | - | 138°C |
| Compound (193) | - | 127°C |
| Compound (198) | - | 142°C |
| Compound (204) | 349°C | 126°C |
| Compound (209) | - | 110°C |
| Compound (214) | - | 124°C |
| Compound (219) | - | 141°C |

Since the arylamine compounds represented by the general formula (a) or (b) have glass transition points of 100°C or higher, it is indicated that the thin film states are stable.

### [Example 18]

Vapor-deposited films with a film thickness of 100 nm were prepared on an ITO substrate by using the arylamine compounds represented by the general formula (a) or (b), and the work functions were measured by an ionization potential measuring device (PYS-202, manufactured by Sumitomo Heavy Industries, Ltd.). The results are shown in Table 2.

**[Table 2]**

| | Work function |
|---|---|
| Compound (1) | -5.69 |
| Compound (38) | -5.70 |
| Compound (81) | -5.75 |
| Compound (130) | -5.72 |
| Compound (198) | -5.70 |
| Compound (11) | -5.69 |
| Compound (54) | -5.67 |
| Compound (93) | -5.73 |
| Compound (142) | -5.67 |
| Compound (183) | -5.67 |
| Compound (188) | -5.69 |
| Compound (193) | -5.74 |
| Compound (204) | -5.65 |
| Compound (209) | -5.66 |
| Compound (214) | -5.70 |
| Compound (219) | -5.67 |

Since the arylamine compounds represented by the general formula (a) or (b) show preferable energy levels compared to the work function of 5.4 eV of a general hole transport material such as NPD, TPD, or the like, they have good hole transport abilities.

### [Example 19]

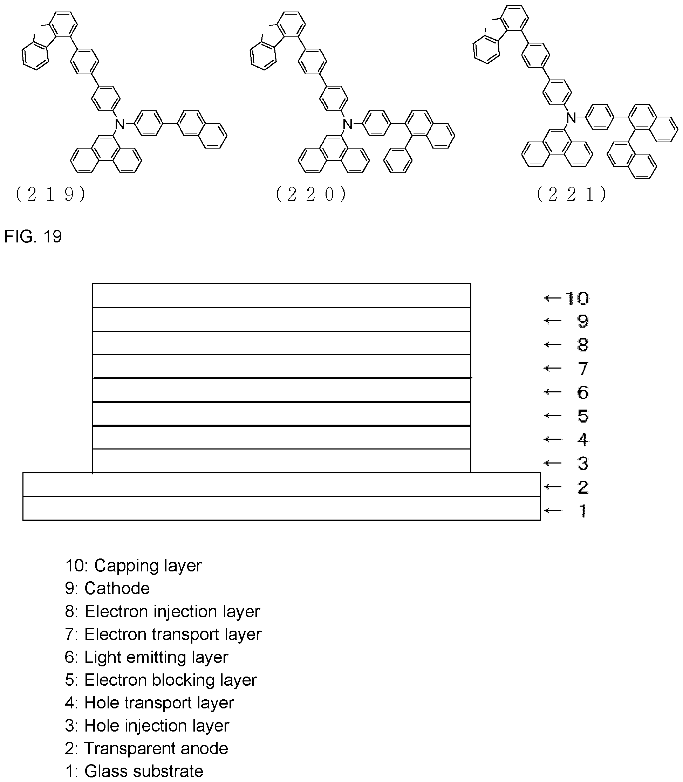

As shown in FIG. 19, the organic EL element was manufactured by depositing a hole injection layer 3, a hole transport layer 4, an electron blocking layer 5, a light emitting layer 6, an electron transport layer 7, an electron injection layer 8, a cathode 9, and a capping layer 10 in that order on a glass substrate 1 on which a reflective ITO electrode had previously been formed as a transparent anode 2.

Specifically, after a glass substrate 1 on which ITO with a film thickness of 50 nm, a silver alloy reflective film with a film thickness of 100 nm, and ITO with a film thickness of 5 nm were sequentially formed was ultrasonic cleaned in isopropyl alcohol for 20 minutes, drying was performed for 10 minutes on a hot plate heated to 250°C. After that, UV ozone treatment was performed for 2 minutes, and then this glass substrate on which ITO was formed was mounted in a vacuum evaporator, and the pressure was reduced to 0.001 Pa or less.

Subsequently, an electron acceptor (Acceptor-1) of the following structural formula and Compound (HTM-1) of the following structural formula as a hole injection layer 3 to cover the transparent anode 2 were subjected to binary deposition at a deposition rate in which a deposition rate ratio becomes Acceptor-1:HTM-1 = 3:97, and thus formed to have a film thickness of 10 nm.

On this hole injection layer 3, Compound (HTM-1) of the following structural formula was formed as a hole transport layer 4 to have a film thickness of 140 nm.

On this hole transport layer 4, Compound (1) of Example 1 was formed as an electron blocking layer 5 to have a film thickness of 5 nm.

On this electron blocking layer 5, Compound (EMD-1) having the following structural formula and Compound (EMH-1) having the following structural formula as the light emitting layer 6 were subjected to binary deposition at a deposition rate in which a deposition rate ratio becomes EMD-1:EMH-1 = 5:95, and thus formed to have a film thickness of 20 nm.

On this light emitting layer 6, Compound (ETM-1) having the following structural formula and Compound (ETM-2) having the following structural formula as the electron transport layer 7 were subjected to binary deposition at a deposition rate in which a deposition rate ratio becomes ETM-1:ETM-2 = 50:50, and thus formed to have a film thickness of 30 nm.

On this electron transport layer 7, lithium fluoride was formed as an electron injection layer 8 to have a film thickness of 1 nm.

On this electron injection layer 8, a magnesium silver alloy was formed as the cathode 9 to have a film thickness of 12 nm.

Finally, Compound (CPL-1) with the following structural formula was formed as the capping layer 10 to have a film thickness of 60 nm.

The properties of the manufactured organic EL element were measured in the air and at room temperature.

Table 3 summarizes and shows the measurement results of the luminescence characteristics obtained by applying a direct current voltage to the manufactured organic EL element.

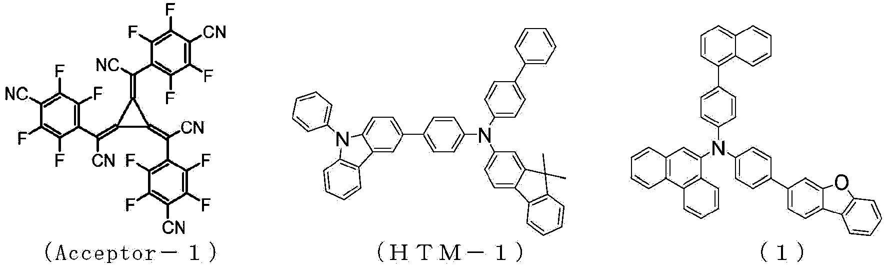

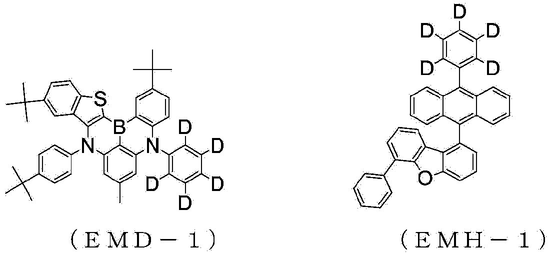

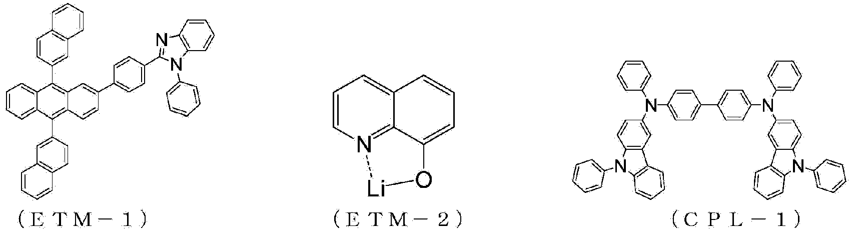

### [Examples 20 to 34]

Organic EL elements were manufactured under the same conditions except that the compounds obtained in Examples 2 to 16, respectively, were used instead of Compound (1) in Example 1 as the material for the electron blocking layer 5 in Example 19. The properties of the manufactured organic EL elements were measured in the air and at room temperature. Table 3 summarizes and shows the measurement results of the luminescence characteristics when a direct current voltage is applied to the manufactured organic EL elements.

### [Comparative Example 1]

For comparison, an organic EL element was manufactured under the same conditions except that Compound (HTM-2) (e.g., see Patent Document 7) of the following structural formula was used instead of Compound (1) in Example 1 as the material of the electron blocking layer 5 in Example 19. The properties of the manufactured organic EL element were measured in the air and at room temperature. Table 3 summarizes and shows the measurement results of the luminescence characteristics when a direct current voltage is applied to the manufactured organic EL element.

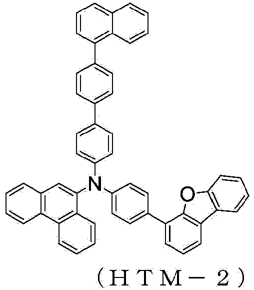

### [Comparative Example 2]

For comparison, an organic EL element was manufactured under the same conditions except that Compound (HTM-3) (e.g., see Patent Document 8) of the following structural formula was used instead of Compound (1) in Example 1 as the material of the electron blocking layer 5 in Example 19. The properties of the manufactured organic EL element were measured in the air and at room temperature. Table 3 summarizes and shows the measurement results of the luminescence characteristics when a direct current voltage is applied to the manufactured organic EL element.

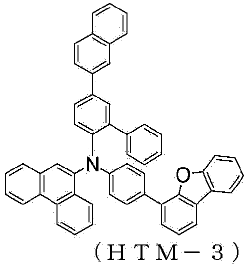

The results of measuring element lifespans using the organic EL elements manufactured in Examples 19 to 34 and Comparative Examples 1 and 2 are summarized and shown in Table 3. When constant current driving was performed with the emission luminance (initial luminance) at the start of emission being 1000 cd/m², the element lifespans were measured as the time until the emission luminance attenuates to 950 cd/m² (equivalent to 95% when the initial luminance is 100%: 95% attenuation).

**[Table 3]**

| | Electron blocking layer | Voltage [V] (@10 mA/cm²) | Luminance [cd/m²] (@10 mA/cm²) | Emission efficiency [cd/A] (@10 mA/cm²) | Power efficiency [Im/W] (@10 mA/cm²) | Element lifespan 95% attenuation |
|---|---|---|---|---|---|---|
| Example 19 | Compound (1) | 3.24 | 1022 | 10.21 | 9.92 | 451 hours |
| Example 20 | Compound (38) | 3.21 | 943 | 9.42 | 9.22 | 510 hours |
| Example 21 | Compound (81) | 3.26 | 1026 | 10.27 | 9.91 | 435 hours |
| Example 22 | Compound (130) | 3.18 | 930 | 9.29 | 9.18 | 489 hours |
| Example 23 | Compound (11) | 3.23 | 1024 | 10.27 | 9.79 | 590 hours |
| Example 24 | Compound (54) | 3.27 | 993 | 9.96 | 9.53 | 439 hours |
| Example 25 | Compound (93) | 3.27 | 1010 | 10.09 | 9.70 | 555 hours |
| Example 26 | Compound (142) | 3.27 | 1027 | 10.29 | 9.86 | 533 hours |
| Example 27 | Compound (183) | 3.26 | 1000 | 10.02 | 9.68 | 444 hours |
| Example 28 | Compound (188) | 3.27 | 998 | 10.01 | 9.61 | 523 hours |
| Example 29 | Compound (193) | 3.26 | 995 | 9.95 | 9.51 | 430 hours |
| Example 30 | Compound (198) | 3.21 | 1015 | 10.18 | 9.57 | 598 hours |
| Example 31 | Compound (204) | 3.21 | 971 | 9.74 | 9.41 | 426 hours |
| Example 32 | Compound (209) | 3.21 | 940 | 9.40 | 9.06 | 485 hours |
| Example 33 | Compound (214) | 3.25 | 1038 | 10.38 | 9.59 | 439 hours |
| Example 34 | Compound (219) | 3.24 | 1022 | 10.24 | 9.61 | 473 hours |
| Comparative Example 1 | HTM-2 | 3.28 | 927 | 9.27 | 8.81 | 355 hours |
| Comparative Example 2 | HTM-3 | 3.31 | 920 | 9.19 | 8.73 | 422 hours |

As shown in Table 3, the luminous efficiencies when a current with a current density of 10 mA/cm² is applied had efficiencies of 9.29 to 10.38 cd/A for the organic EL elements of Examples 19 to 34, which were equivalent or higher than 9.19 to 9.27 cd/A for the organic EL elements of Comparative Examples 1 and 2. Also, regarding power efficiency, the organic EL elements of Examples 19 to 34 had high efficiencies of 9.06 to 9.92 Im/W compared to 8.73 to 8.81 Im/W for the organic EL elements of Comparative Examples 1 and 2. In addition, it can be seen that the element lifespans (95% attenuation) of the organic EL elements of Examples 19 to 34 are increased to 426 to 598 hours compared to 355 to 422 hours for the organic EL elements of Comparative Examples 1 and 2.

As is clear from the above results, since the organic EL element of the present disclosure uses an arylamine compound with high hole mobility and excellent electron blocking ability, it could be found that it has high luminous efficiency compared to the conventional organic EL element, and a long-lifespan organic EL element can be realized.

### [Explanation of reference numerals]

1: Glass substrate
2: Transparent anode
3: Hole injection layer
4: Hole transport layer
5: Electron blocking layer
6: Light emitting layer
7: Electron transport layer
8: Electron injection layer
9: Cathode
10: Capping layer

### [Industrial Applicability]

The organic EL element using the arylamine compound having the specific structure of the present disclosure can improve the durability of the organic EL element along with improved luminous efficiency, and for example, it has become possible to deploy it in home telephone products and lighting applications.

## Claims

1. An arylamine compound represented by the following general formula (a) or (b): 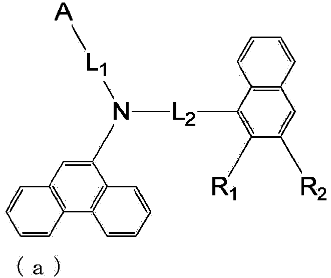 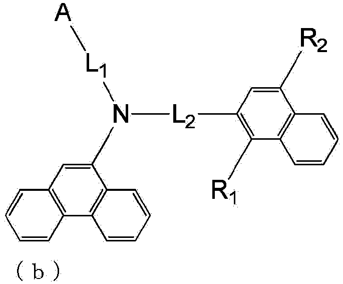 wherein
A indicates a substituted or unsubstituted dibenzofuranyl group, or a substituted or unsubstituted dibenzothienyl group,
L₁ indicates a divalent group obtained by removing two hydrogen atoms from unsubstituted benzene, or a divalent group obtained by removing two hydrogen atoms from unsubstituted biphenyl,
L₂ indicates a divalent group obtained by removing two hydrogen atoms from unsubstituted benzene, and
R₁ and R₂ are identical or different and respectively indicate a hydrogen atom, a substituted or unsubstituted aromatic hydrocarbon group, a substituted or unsubstituted aromatic heterocyclic group, or a substituted or unsubstituted condensed polycyclic aromatic group.

2. The arylamine compound of claim 1, represented by the following formula (c), (d), (e), or (f): 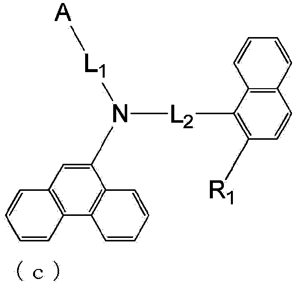 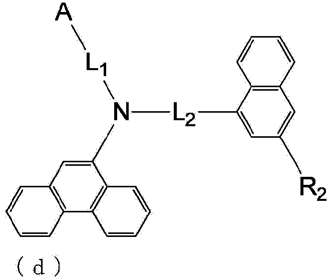 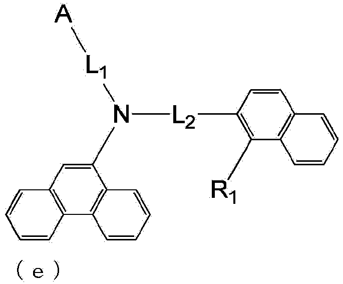 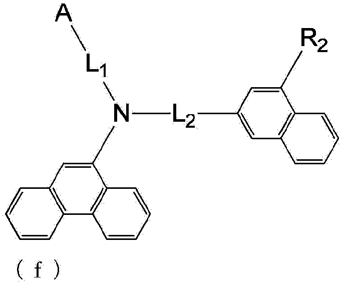 wherein A, L₁, L₂, R₁, and R₂ are the same definitions as in the general formula (a) or (b).

3. The arylamine compound of claim 1 or 2, wherein L₂ in the general formula (c), (d), (e), or (f) above is represented by a 1,4-phenylene group.

4. The arylamine compound of any one of claims 1 to 3, wherein A in the general formula (c), (d), (e), or (f) above is represented by an unsubstituted dibenzofuranyl group or an unsubstituted dibenzothienyl group.

5. The arylamine compound of any one of claims 1 to 4, wherein A in the general formula (c), (d), (e), or (f) above is represented by an unsubstituted dibenzofuranyl group.

6. The arylamine compound of any one of claims 1 to 5, wherein A in the general formula (c), (d), (e), or (f) above is represented by an unsubstituted 3-dibenzofuranyl group or an unsubstituted 4-dibenzofuranyl group.

7. An organic EL element having a pair of electrodes and at least one organic layer sandwiched between them, wherein the organic layer contains the arylamine compound of any one of claims 1 to 6.

8. The organic EL element of claim 7, wherein the organic layer is a hole transport layer.

9. The organic EL element of claim 7, wherein the organic layer is an electron blocking layer.

10. The organic EL element of claim 7, wherein the organic layer is a hole injection layer.

11. The organic EL element of claim 7, wherein the organic layer is a light emitting layer.

12. An electronic device having a pair of electrodes and at least one organic layer sandwiched between them, wherein the organic layer contains the arylamine compound described in any one of claims 1 to 6.
